# EUROPEAN PATENT APPLICATION

(11) **EP 1 832 875 A1**
(43) Date of publication of application: **12.09.2007**
(21) Application number: 04821651.9
(22) Date of filing: 28.12.2004
(51) Int. Cl.: G01N 33/543, G01N 33/483, C07K 17/04

(54) **BIOLOGICAL MICROCHIP FOR MULTIPLE PARALLEL IMMUNOASSAY OF COMPOUNDS AND IMMUNOASSAY METHODS USING SAID MICROCHIP**

(71) Applicant: INSTITUT MOLEKULYARNOI BIOLOGII IM. V.A.ENGELGARDTA ROSSIISKOI AKADEMII NAUK, Moscow, 117984 (RU)
(72) Inventor: DARY, Ekaterina Lvovna, Moscow, 107078 (RU); DEMENTIEVA, Ekaterina Igorevna, Moscow, 117393 (RU); BUTVILOVSKAYA, Veronika Igorevna, Moscow, 121165 (RU); ZASEDATELEV, Alexandr Sergeevich, Moscow, 117418 (RU); RUBINA, Alla Yurievna, Moscow, 117313 (RU); STOMAKHIN, Andrei Alexandrovich, Moscow, 117461 (RU); SAVVATEEVA, Elena Nikolaevna, Moscow, 119607 (RU)
(74) Representative: Boije-Backman, Solveig Magdalena
(86) International application number: PCT/RU2004/000524
(87) International publication number: WO 2006/071132

(57) **Abstract**

The present invention relates to biochemistry, medicine, and molecular biology, in particular to analytical biochemistry and immunochemical assay, and is concerned with a biological microchip for multiple parallel detection and quantitative determination of different compounds. The biochip comprises an array of three-dimensional hydrogel elements which have a predetermined volume, formed on a support by the method of photo- or chemically induced polymerization, and containing biological molecules of the same or different nature (ligands). The invention provides a method for detecting compounds on the biological microchip, comprising identification thereof by mass spectrometry techniques directly on the hydrogel element of the microchip and a method for detecting immunoassay results, consisting in carrying an immuno-PCR and registering the results thereof on the same microchip. The invention also relates to a method of multiple parallel immunoassay of compounds on a biological microchip. Biological microchips for immunoassay and a method of carrying out multiple parallel analysis of compounds can find application in analyzing a wide range of high-molecular and low-molecular compounds, in medicine, pharmacology, food industry, environmental protection, in research work, particularly in proteomics.

## Description

### Field of the Art

The present invention relates to biochemistry, medicine, and molecular biology, in particular to analytical biochemistry and immunochemical assay, and is concerned with multiple parallel detection and quantitative determination of different compounds by an immunochemical method using three-dimensional hydrogel-based microchips.

The proposed biological microchips for immunoassay and methods for carrying out multiple parallel assay of compounds may find application in different fields of science and technology for the determination of a wide range of low-molecular and high-molecular compounds, such as markers of various diseases, allergens, hormones, physiologically active substances in medicine, pharmacology, food industry, environmental protection, in research work, particularly for the determination of proteins in proteomics.

### State of the Art

At present, immunochemical methods of assay have found wide application in analytical biochemistry. Most widespread are solid-phase methods using antibodies adsorbed or immobilized in the wells of immunological plates. For detecting the results of assay, use is made of radioactively labeled compounds (radioimmunoassay (RIA)), of fluorescently labeled compounds, and also of enzymes (e.g., ELISA technique).

The advantages offered by an immunoassay on plates are: high sensitivity of detection (pico- or nanogramms of the compound under analysis in 1 ml of the investigated solution), possibility of automating the assay (automation of the steps of adding reagents, washings and simultaneous registration of signals from each of the plate wells) (A.M. Egorov, A.P. Osipov, B.B. Dzantiev, E.M., Gavrilova (Eds.), Theory and Practice of Enzyme-linked Immunoassay Assay. Moscow: Vysshaya Shkola. 1991 (in Russian)).

A disadvantage of conventional immunological methods is that a sample cannot be tested simultaneously for the presence of several compounds. The use of microchips- arrays of individual cells containing various probes (proteins, receptors, antibodies, antigens, etc.) - opens up a new stage in analytical biochemistry The main advantage of using microchips is that hundreds or thousands of various probes can be immobilized in the cells of one microchip and, in contrast to conventional methods, the possibility of simultaneous quantitative assay of numerous substances on one microchip is realized. Microchips allow carrying out multiple parallel assay of samples, whereby the assay efficiency is materially enhanced, carrying out research work can be miniaturized, and the required amount of material to be investigated is reduced.

Several variants of protein microchips employed for carrying out immunoassays have been described, for instance, arrays where separate elements are applied on the surface of glass, plastic, etc., microchips in the form of microwells or microjet elements (P. Angenendt, J. Glökler, D. Murphy, H. Lehrach, D.J. Cahill, Toward optimized antibody microarrays: a comparison of current microarray support materials, Anal. Biochem., 2002, 309, 253-260; J. Glökler, P. Angenendt, Protein and microarray technology, J. Chromatogr. B, 2003, 797, 229-240; S. Seong, C. Choi, Current status of protein chip development in terms of fabrication and application, Proteomics, 2003, 3, 2176-2189).

In most cases protein chips are two-dimensional, i.e., proteins are located on the surface of the carrier; the proteins can be adsorbed by or covalently bound to the support. For covalent immobilization, the surface of the carrier (glass, membranes, etc.) is subjected to chemical treatment for introducing amino-, aldehydo-, sulfhydryl- or epoxy groups. For increasing the amount of protein bound to the surface of the carrier, various spacers have been proposed (P. Angenendt, J. Glökler, D. Murphy, H. Lehrach, D.J. Cahill, Toward optimized antibody microarrays: a comparison of current microarray support materials, Anal. Biochem., 2002, 309, 253-260; J. Glökler, P. Angenendt, Protein and microarray technology, J. Chromatogr. B, 2003, 797, 229-240; S. Seong, C. Choi, Current status of protein chip development in terms of fabrication and application, Proteomics, 2003, 3, 2176-2189; H. Zhu, M. Snyder, Protein chip technology, Curr. Opin. Chem. Biol., 2003, 7, 55-63). Among the disadvantages of two-dimensional chips are: denaturation of proteins on the surface of the carrier and at the interface, leading to the loss of functional activity by the proteins and, as a consequence, low sensitivity of the assay.

Three-dimensional microchips based on polyacrylamide hydrogels, containing immobilized DNA and protein molecules, were developed for the first time at IMB RAS (G.M. Ershov, A.D. Mirzabekov, Method of manufacturing a matrix for the detection of mismatches, US Patent No. 5770721). The technology of manufacturing those microchips consisted of several steps, comprising: (1) preparing a support, (2) forming a matrix of gel cells thereon, (3) applying solutions of biological macromolecules to the cells in accordance with a preliminarily drawn scheme of the biochip, (4) chemical treatment of the cells with to allow immobilization of probe molecules, (5) washing and drying the resulting microchips (G.M. Ershov, A.D. Mirzabekov, Method of manufacturing a matrix for the detection of mismatches, US Patent No. 5770721; D. Guschin, G. Yershov, A. Zaslavsky, A. Gemmell, V. Shick, D. Proudnikov, P. Arenkolv, A. Mirzabekov, Manual manufacturing of oligonucleotide, DNA, and protein microchips, Anal. Biocxhem, 1997, 250, 203-211).

A disadvantage of those microchips was that they were manufactured by using a complicated and inefficient technology, which did not provide uniformity of the coating and immobilization of probes, and the probes (proteins or DNAs) were immobilized, mainly, on the gel surface. These disadvantages were overcome in the development of gel microchips of subsequent generations, manufactured by copolymerization and polymerization immobilization techniques (A.D. Mirzabekov, A.Yu. Rubina, S.V. Pan'kov, B.K. Chernov, Method of immobilization of oligonucleotides containing unsaturated groups in polymeric hydrogels in forming microchip, Russian Patent No. 2175972, November 20, 2001 (Bull. Izobret., 2001, No. 25); A.D. Mirzabekov, A.Yu,. Rubina, S.V. Pan'kov, Method of polymerization immobilization of biological molecules and composition for its realization RF Patent No. 2216547 (B.I.P.M. No. 32, 20.11.2003). International Application PCT/RU 01/004204, WO 03/033539).

The Biocept Company had patented polyurethane-based hydrogel microchips, which, in particular, were used for the immobilization of antibodies and carrying out immunoassay (Method of making biochips and the biochips resulting therefrom. US Patent 6,174,683, Jan. 16, 2001).

For the biochips manufactured by the Biocept Company there is shown only the principle possibility of using thereof in immunoassay, but no particular examples and results of the assay (sensitivity, reproducibility, etc.) are given.

Chips of different construction for immunological detection of various diseases, allergens, cytokines, etc., have been described, for instance, two-dimensional microchips containing antibodies sorbed on Hybond membranes (R.P. Huang, R. Huang, Y. Fan, Y. Lin, Simultaneous detection of multiple cytokines from conditioned media and patient's sera by an antibody-based protein array system, Anal. Biochem., 2001, 294, 55-62) were used for immunochemical detection of 24 cytokines in blood sera by sandwich-type immunoassay.

After incubating membranes with biological samples, microchips were developed using conjugates of secondary antibodies with biotin and streptavidin peroxidase. Peroxidase-catalyzed chemiluminescence signals were recorded.

In the investigations of Haab et al. antibody-containing microchips for the identification and assay of tumor-associated markers are described (J.C. Miller, H. Zhou, J. Kwekel, R. Cavallo, J. Burke, E.B. Butler, B.S. Teh, B.B. Haab, Antibody microarray profiling of human prostate cancer sera, Proteomics, 2003, 3, 56-63).

Microchips were manufactured on two supports: glass treated with poly-L-lysine and polyacrylamide-based hydrogel. The microchips contained 184 different antibodies. Fluorescent signals from polyacrylamide gel-based microchip elements were 6 times higher than from two-dimensional microchip elements. This demonstrated an advantage of the immobilization in the gel layer volume over the immobilization on the two-dimensional surface, even if the latter is modified.

For determining specific IgE in allergy assay a microchip has been proposed - a glass slide to which 94 different most widespread allergens are applied (R. Hiller, S. Laffer, C. Harwanegg, M. Huber, W.M. Schmidt, A. Twardosz, B. Barletta, W.M. Becker, K. Blaser, H. Breiteneder, M. Chapman, R. Crameri, M. Duchene, F. Ferreira, H. Fiebig, K. Hoffmann-Sommergruber, T.P. King, T. Kleber-Janke, V.P. Kurup, S.B. Lehrer, J. Lidholm, U. Muller, C. Pini, G. Reese, O. Scheiner, A. Scheynius, H.D. Shen, S. Spitzauer, R. Suck, The I. Swoboda, W. Thomas, R. Tinghino, M. Van Hage-Hamsten, T. Virtanen, D. Kraft, M.W. Muller, R. Valenta, Microarrayed allergen molecules: diagnostic of gatekeepers for allergy of treatment, FASEB J., 2002, 15, 414-416).

Purified allergen proteins, both recombinant and from natural sources, and also peptides and low-molecular compounds were used. 40 µl of blood serum were required for the assay. After the interaction of the microchip with the blood serum, the chip was treated with fluorescently labeled antibodies against human IgE. A good correlation was observed between the intensity of fluorescent signals on the microchip from the cells containing corresponding specific IgE, and the results of the measurements of the same blood sera by standard methods (EL1SA- test, immunoblot, and others).

For characterizing the quality of blood preparations, for determining the blood group, the compatibility of donor and recipient microchips with immobilized oligosaccharides can be used. In the work of O.E. Galanina, M. Mecklenburg, N. E. Nifantiev, G. V. Pazynina, N. V. Bovin, GlycoChip: multiarray for the of study of carbohydrate-binding of proteins, Lab Chip, 2003, 3, 260-265, microchips with immobilized oligosaccharides are proposed, obtained on a standard support for DNA-microchips XNAonGOLD^{™} of Inter-activa Biotechnologie GmbH (Germany).

Solutions of biotinylated conjugates of oligosaccharides with 30 kDa polyacrylamide were applied with the help of a pipette to a microscope slide covered with streptavidin containing 2 x 96 cells (1 µl per cell). The microchips were used for investigating the specificity of antibodies to carbohydrate antigens. The obtained results were consistent with the results obtained by the standard ELISA method on plates.

For detecting the results of immunoassay on the microchips, fluorescence is most frequently used (detection on fluorescent microscopes, scanners, confocal microscope), chemi- and bioluminescence (with the introduction of enzymatic labels, such as peroxidase, alkaline phosphatase, luciferase and others), mass spectrometry.

For amplifying the signal in carrying out sandwich-type immunoassay, the authors (T. Sano, C.L. Smith, C.R. Cantor, Immuno-PCR: very sensitive antigen detection by means of specific antibody-DNA conjugates, Science, 1992, 258, 120-122) proposed the method of immuno-PCR. Assay was carried out in a 96-cell plate, the PCR result was further evaluated by electrophoresis data. It was shown by the authors, that the use of PCR for the immunoassay gives better results than the classic enzyme-linked immuno- and even radioimmunoassay ((T. Sano et al., supra; E.R. Hendrickson, T.M. Truby, R.D. Joerger, W.R. Marjarian, R.C. Ebersole, High sensitivity multianalyte immunoassay using covalent DNA-labeled antibodies and polymerase chain reaction, Nucleic Acids Res., 1995,23,522-529).

One more variant of signal amplification is the rolling circle amplification, RCA method proposed in 2000 for assay in the 2D microchip format (B. Schweitzer, S. Wiltshire, J. Lambert, S. O'Malley, K. Kukanskis, Z. Zhu, S. Kingsmore, P.M. Lizardi, D.C. Ward, Immunoassays with rolling circle DNA amplification: a versatile of platform for ultrasensitive antigen detection. Proc. Natl. Acad. Sci. USA, 2000, 97, 10113-10119).

Immunoassay with mass-spectral detection is described for chips produced by Ciphergen Biosystems (USA). This company has constructed chips and an instrument for the assay of proteins directly from the chips by the method of SELDI mass spectrometry (T.W, Hutchens, T.- T. Yip, Surface-enhanced neat desorption for disorption and detection of analytes. USA patent No. 5,894,063).

These chips are aluminum strips with the size of approximately of 8x 1 cm, containing 8 separate identical elements for applying samples - affine carriers for the specific binding of proteins. In the production of a microchip for the immunoassay of prostate-specific membrane antigen (PSMA) antibodies were immobilized on the preliminarily activated surface of the aluminum strip. After the incubation with blood serum and formation of the antibody-PSMA complex, the bound PSMA was determined with the aid of SELDI mass spectrometry (Z. Xiao, B.L. Adam, L.H. Cazares, M.A. Clements, J.W. Davis, P.F. Schellhammer, E.A. Dalmasso, G.L. Wright, Quantitation of serum prostate-specific membrane antigen by a novel protein biochip immunoassay discriminates benign malignant prostate disease, Cancer Res., 2001, 61, 6029-6033).

The chips (strips) proposed by the Ciphergen Biosystems company are two-dimensional, the specific absorption of proteins occurs on the carrier surface. Each aluminum strip makes it possible to analyze only 8 samples, and said elements, strictly speaking, are not microchips.

The duration of immunochemical assay depends on the rate of formation of antigen-antibody complexes, and in the case of microchips the diffusion of the antigen (antibody) to the antibodies (antigens) immobilized in the microchip cells can be the limiting factor. Agitating the sample is one of the methods of accelerating the assay. Acceleration of the reaction was observed, for example, when agitating a sample on two-dimensional DNA-microchips (A. Toegl, R. Kirchner, S. Gauer, A. Wixforce, Enhancing results of microarray hydridizations through microagitation, J. Biomol. Techniques, 2003, 4, 197-204).

The microchip (microscope slide) for carrying out the reaction with agitation was secured to a specially designed camera (AdvaCard). The agitation was accomplished with the aid of acoustic waves. The employed volume of the sample was 10 µl and less. It was noted that as a result of the agitation the reaction time decreased, the signal/background ratio increased, and the reproducibility of the results improved.

### Disadvantages

In the known methods of carrying out immunoassay on microchips mainly two-dimensional microchips are used, in which antibodies or antigens are applied on the surface of the carrier (glass, plastic, a membrane etc.). The disadvantages of two-dimensional microchips is low and uncontrollable effectiveness of immobilization, the presence of contact of the immobilized compounds with the hydrophobic surface of the carrier, which leads to loss of the biological activity of probes, especially of the probes of protein nature, and, accordingly, to decrease of the immunoassay sensitivity. For the majority of immunological microchips, with an increase in the number of immobilized antibodies, the sensitivity of assay and reproducibility of results sharply falls (coefficient of variation ~50%) (B. Schweitzer, S.F. Kingsmore, Measuring of proteins on microarrays. Curr. Opin. Biotechnol, 2002, 13, 14-19).

Among the disadvantages of microchips of another construction (micro-wells, electronic microchips, microjet elements etc.) is complicated technology of manufacture and registration of signals with the use of complicated and expensive equipment (confocal microscope, Raman spectroscopy, etc.) For registering signals by mass spectrometry techniques only chips produced by the Ciphergen Biosystems (USA) for the assay by the SELDI MS method, consisting of 8 elements, with a large size of cells have been proposed.

The disadvantages of the microchips known from the state of the art and of the methods of immunoassay in which they are used are overcome by the present invention, which provides methods for the immunoassay of compounds with the use of three-dimensional microchips based on hydrogels.

### Disclosure of the Invention

The first aspect of the invention is a biological microchip for multiple parallel immunoassay of compounds, said microchip comprising an array of three-dimensional hydrogel elements of a prescribed volume, formed on a support by the method of photo- or chemically induced polymerization, and containing biological molecules of the same or different nature (ligands). Said biological microchip can simultaneously contain cells with immobilized proteins, enzymes, antibodies, polysaccharides, DNA, RNA and low-molecular compounds, in each separate cell an individual compound being immobilized. This biological microchip allows sequential carrying out reactions of different types, for example, sequential interaction of an antigen with an antibody (immunological reaction) and of an enzyme with substrates (enzymatic reaction) with the use of conjugates of antibodies or antigens with enzymes. In another embodiment there are contemplated sequential interaction of an antigen with an antibody (immunological reaction), polymerase chain reaction (PCR) on the microchip with the use of conjugates of antibodies or antigens with oligonucleotides and hybridization with the formation of a specific complex between the oligonucleotides obtained as a result of PCR with the complementary oligonucleotides immobilized in other cells of the same microchip.

Three-dimensional hydrogel elements of the microchip are covalently fixed on the surface of the support (glass, silicon or plastic) and are separated from each other by the hydrophobic surface of the support. In each gel element an immobilized ligand of protein or non-protein nature is contained, which during the microchip incubation in the reaction medium including a sample comprising compounds subject to assay forms a specific immune complex of "antigen-antibody" type. In this step separation of the compounds under analysis from the mixture takes place according to the ability of compounds to bind specifically the immobilized ligands, whereby it is possible to carry out simultaneous parallel assay of several objects on one microchip.

Three-dimensional gel elements have a considerably greater capacity in comparison with the elements of two-dimensional microchips, whereby the assay sensitivity is increased. The hydrophilic environment of the ligands immobilized in the hydrogel structure stabilizes the structure of the ligands, which enables them to completely preserve their biological activity.

The ligands immobilized in the hydrogel cells of the biological microchip are selected from the group comprising antibodies of any type and antigens of different nature.

As objects of investigation for the quantitative immunological assay according to the invention there are preferably proposed:
- tumor-associated antigens which are markers of a number of oncological diseases;
- total human immunoglobulin E;
- specific antibodies (immunoglobulins E) to different allergens;
- antibodies to different polysaccharides;
- different polysaccharides.

For the immunoassay of tumor-associated antigens - markers of oncological diseases, antibodies to said tumor-associated antigens and/or tumor-associated antigens are used as immobilized ligands. For the immunological determination of total human immunoglobulin E, for example, when determining the immune status of organism, antibodies against human immunoglobulins E and/or human immunoglobulins E are used as the immobilized ligands of the biological microchip. For the immunoassay of specific antibodies to different allergens, allergens are used as immobilized ligands of the biological microchip. When biological microchip is used for the immunoassay of specific antibodies to polysaccharides and of polysaccharides, different polysaccharides and antibodies against polysaccharides are employed as immobilized ligands.

The second aspect of the invention is a method of detecting compounds on the biological microchip according to the first aspect of the invention, including identification of compounds by the method of mass spectrometry, characterized by that the identification is carried out directly on the hydrogel element of the microchip through the results of direct mass-spectral assay of the compound bound to the ligand. A procedure of obtaining MALDI TOF mass spectra directly from the gel cells of the microchip has been developed. In the course of interaction of the microchip with the reaction medium containing the compounds under analysis a complex is formed between the ligand immobilized on the biological microchip and the compound under analysis. After elution under the conditions destroying the complex, direct mass spectral assay is carried out from each gel element, and the compound under analysis is identified by its molecular weight and ability to bind to the definite ligand.

The next aspect of the invention is a method of detecting compounds of protein nature on the biological microchip according to the first aspect of the invention, including their identification by the method of mass spectrometry, characterized by that the identification is carried out directly on the hydrogel element of microchip through the results of the direct mass-spectral assay of the protein bound to the ligand and/or peptides obtained by carrying out proteolytic hydrolysis of the protein bound to the ligand directly on the hydrogel element of the microchip.

A still further aspect of the invention is a method of multiple parallel immunoassay of compounds on the biological microchip, comprising carrying out several steps:
*a)* incubation of the biological microchip in a reaction medium comprising a sample containing compounds under analysis for forming immune complexes with the ligands immobilized on the microchip, which incubation, if necessary, is carried out under agitation conditions;
*b*) detection of the formed complex;
c) if necessary, quantitative analysis of the compound under analysis.

In the first step of the assay incubation of the biological microchip in a reaction medium is carried out to form complexes of the "antigen-antibody" type. If necessary, this step is carried out under agitation conditions, whereby carrying out of the immunoassay becomes appreciably accelerated.

The method of multiple parallel immunoassay of compounds on the biological microchip can be realized in any format (direct, indirect, competitive, sandwich-type immunoassay, and others).

In the case of direct immunoassay the reaction medium in step *a)* contains the compound to be analyzed, which forms a specific complex with the antibodies against this compound, immobilized on the chip. The higher is the concentration of the compound under analysis in the sample, the is higher the signal, recorded from the corresponding gel cells of the microchip.

Competitive assay is carried out on microchips with immobilized antibodies or on microchips with immobilized antigens, for example, with compounds under analysis. In one of the embodiments to the reaction medium in step *a)* the compound under analysis containing a label is further added in a definite concentration. A competition between the labeled and unlabeled compound for binding to the antibodies immobilized on the biological microchip occurs. In another embodiment of the competitive assay the reaction medium in step *a)* additionally contains labeled antibodies against the compound under analysis, and there takes place competition of the labeled antibodies for binding to the compound under analysis in solution and the compound immobilized on the microchip. In the case of competitive assay, the higher is the signal registered from the corresponding cells of the microchip, the smaller is the concentration of the compound under analysis in the sample.

The methods of direct and competitive immunoassay are possible both for compounds of protein nature and for low-molecular compounds. The sandwich-type variant of immunoassay is possible for compounds of protein nature, for which antibodies specific to different epitopes of protein molecule are accessible.

In the case of the sandwich-type immunoassay the biological microchip contains immobilized antibodies against the compound under analysis, the reaction medium in step *a)* contains the compound under analysis, and the formation of specific antigen - immobilized antibody complex takes place. After the formation of the complex the microchip is developed with the aid of labeled antibodies, specific to another epitope of the compound under analysis. For the sandwich-type immunoassay a variant is also possible, when the reaction medium in step *a)* additionally contains developing antibodies, which reduces the assay time, since incubation of the microchip with the compound under analysis and development occur simultaneously. The higher is the concentration of the compound under analysis in the sample, the higher is the signal recorded from the corresponding gel cells of the microchip.

The registration of the immunoassay results is carried out with the use of fluorescence, chemiluminescence, mass-spectrometry directly from the gel elements of the microchip. For the quantitative analysis of the compound under analysis in step *c)*, steps *a)-b)* are carried out with standard (reference) samples containing known concentrations of the compound under analysis, and a calibration curve "signal vs. comcentration of compound under analysis" is plotted, using which the content of the compound under analysis in the sample is determined. Calibration curve can also be obtained with the use of ligands immobilized on the microchip. For example, the microchip contains gel elements with immobilized antibodies against the antigen being analyzed and gel elements with the immobilized antigen in different concentrations.

After carrying out the standard sandwich-type immunoassay, i.e., after the incubation of the microchip with the sample and the labeled developing antibodies, the signals obtained from the gel elements with the known concentrations of the immobilized antigen serve for plotting the calibration curve for determining the unknown concentration of the antigen in the sample. Thus, the microchip simultaneously contains the calibration curve and serves for determining the unknown concentration of the antigen in the sample.

The detection of the results of immunoassay is achieved fluorimetrically, chemiluminometrically, mass-spectrometrically or by carrying out immuno-PCR with the subsequent registration of the PCR results on the same microchip. Antibodies and other compounds, used for the detection can contain fluorescent markers or be conjugates with proteins or other compounds, capable of participating in the reactions, leading to the emission of light (chemi- or bioluminescence). Introducing additional markers is not required for mass-spectral detection.

In the case of detection by carrying out immuno-PCR, biological microchip contains simultaneously in different gel cells immobilized ligands for carrying out immunoassay (antibodies or antigens) and oligonucleotides. In the course of interaction of the microchip with the reaction medium containing the compounds under analysis, there takes place formation of a complex between the ligand immobilized on the biological microchip and the compound under analysis. As the label for the detection of the formed complex, an oligonucleotide complementary to the oligonucleotide immobilized in the gel cells is used. Further, PCR is carried out directly on the biological microchip with a fluorescently labeled primer, and the resultant fluorescently labeled PCR-product forms a specific complex with the complementary oligonucleotides immobilized in other cells of the microchip. With the use of this method a significant growth of the signal occurs in comparison with the usual fluorescent detection, since in the course of PCR the fluorescently labeled oligonucleotide is generated in an amount which is several orders of magnitude greater than the initial one, whereby the detection sensitivity is increased markedly.

The method of the present invention makes it possible to carry out multiple parallel immunoassay of several compounds. The biological microchip for the parallel multiple immunoassay simultaneously contains cells with immobilized antibodies against all compounds under analysis and/or other immobilized compounds, in each separate gel cell an individual compound being immobilized. After the incubation of the microchip in the reaction medium including a sample containing several compounds under analysis, specific immune antigen - antibody complexes are formed, and simultaneous detection of the formed complexes in the corresponding cells of the microchip is carried out. In the case of sandwich-type immunoassay and after the incubation in the reaction medium comprising a sample, development is carried out with a mixture of labeled antibodies against all compounds under analysis. A variant is also possible, when the reaction medium in the step *a)* simultaneously contains the compounds under analysis and the labeled developing antibodies.

The sensitivity of the proposed multiple parallel immunoassay of compounds on the biological microchip did not inferior to the sensitivity of the standard variant of immunoassay on plates (strips), for example, the limit of detecting the prostate-specific antigen (marker of prostate cancer) by the sandwich-type immunoassay with the use of gel microchips was 0.05 ng/ml (Table 1).

The next aspect of the present invention is a method of multiple parallel immunoassay of oncomarkers on the biological microchip according to the first aspect of the invention with microchip containing immobilized antibodies against oncomarkers and/or immobilized oncomarkers, contemplating:
*a)* incubation of the biological microchip in a reaction medium comprising a sample, containing oncomarkers under analysis for the formation of an immune complex with ligands immobilized on the microchip, which incubation, if necessary, can be carried out under agitation conditions;
*b)* detection of the formed complex;
*c)* quantitative analysis of the oncomarker under analysis.

Different embodiments of the method can comprise immunoassay of any type (direct, indirect, competitive, sandwich-type immunoassay and others). For the quantitative analysis of an oncomarker in step *c)*, steps *a)-b)* are carried out with the known concentrations of the oncomarker, a calibration curve is plotted for determining the content of the oncomarker under analysis in the sample. The detection of the formed complex in step *b)* is carried out fluorimetrically, chemiluminometrically, mass-spectrometrically, or by carrying out immuno-PCR, followed by registration of the PCR results on the same microchip.

A variant is also possible, when the microchip simultaneously with immobilized antibodies against oncomarkers contains gel elements with immobilized oncomarkers in different concentrations for plotting the calibration curve. After the incubation of the microchip with the sample containing an oncomarker in an unknown concentration, and labeled antibodies against the oncomarker under analysis (sandwich-type immunoassay), the dependence of the signal vs. oncomarker concentration is obtained from the gel elements with the known concentration of the immobilized oncomarker and a calibration curve is plotted, which is used for determining the unknown concentration of the oncomarker in the sample.

A still further aspect of the present invention is a method of immunoassay of total human immunoglobulin E on the biological microchip according to the first aspect of the present invention. Microchip contains immobilized antibodies against human immunoglobulins E (anti-IgE) or immobilized human immunoglobulins E (IgE) and the method contemplates:
*a)* incubation of the biological microchip in a reaction medium comprising a sample, containing total human immunoglobulins subject to analysis for the formation of an immune complex with ligands immobilized on the microchip, which incubation, if necessary, can be carried out under agitation conditions;
*b)* detection of the formed complex;
*c)* quantitative analysis, if necessary, of the total human immunoglobulin E under analysis.

Different embodiments of the method can comprise an immunoassay of any type (direct, indirect, competitive, sandwich-type and others). For the quantitative analysis of total human immunoglobulin E in step *c),* steps *a)-b)* are carried out with the known concentrations of the total human immunoglobulin E under analysis, a calibration curve is plotted for determining the content of the total immunoglobulin E under analysis in the sample. The detection of the formed complex in step *b)* is carried out fluorimetrically, chemiluminometrically, mass-spectrometrically, or by carrying out immuno-PCR, followed by registration of the PCR results on the same microchip.

The calibration dependence for determining the total immunoglobulin E can be also obtained directly on the microchip, if we use a microchip containing gel elements with the immobilized IgE in different concentrations. After the incubation of the microchip with the sample containing IgE in an unknown concentration, and with second labeled antibodies against IgE (sandwich-type immunoassay) from the gel elements with the immobilized IgE the dependence "signal vs. IgE concentration" is obtained, which is used as the calibration curve for determining the unknown concentration of IgE.

Yet another aspect of the present invention is a method of multiple parallel immunoassay of allergen-specific immunoglobulins E on the biological microchip according to the first aspect of present invention with biochip containing immobilized specific allergens, said method contemplating:
*a)* incubation of the biological microchip in a reaction medium comprising a sample, containing allergen-specific IgE under analysis for the formation of an allergen - allergen-specific antibody complex, which incubation, if necessary, is carried out under agitation conditions;
*b)* detection of the formed complex;
*c)* quantitative analysis, if necessary, of the allergen-specific IgE under analysis.

In different embodiments said method can comprise an immunoassay of any type (direct, indirect, competitive, sandwich-type and others). For the quantitative analysis of allergen-specific IgE in step *c),* steps *a)-b)* are carried out with the known concentrations of allergen-specific IgE and a calibration dependence curve is plotted for determining the content of the allergen-specific IgE being analyzed in the sample. Detection of the formed immobilized allergen - allergen-specific antibody complex in step *b)* is carried out fluorimetrically, chemiluminometrically, mass-spectrometrically or by carrying out the immuno-PCR, followed by registration of the PCR results on the same microchip.

For the quantitative analysis of the allergen-specific IgE a microchip is also used, which simultaneously with immobilized allergens contains gel elements with immobilized allergen-specific IgE in different concentrations for plotting the calibration dependence curve. After the incubation of the microchip with the sample containing an allergen-specific IgE in an unknown concentration, and with the labeled indicating antibodies (sandwich-type immunoassay), from the gel elements with the immobilized allergen-specific IgE in the known concentrations a signal vs. analyzed antigen concentration dependence is derived, which is used for determining the unknown concentration of the antigen in the sample.

A still further aspect of the present invention is a method of multiple parallel immunoassay of antibodies against polysaccharides on the biological microchip according to the first aspect of the present invention, the microchip containing immobilized polysaccharides or antibodies against polysaccharides, said method contemplating:
*a)* incubation of the biological microchip in a reaction medium comprising a sample containing antibodies against polysaccharides under analysis for the formation of a polysaccharide-antibody complex, which incubation, if necessary, can be carried out under agitation conditions;
*b)* detection of the formed complex;
*c)* if necessary, quantitative analysis of the antibodies against polysaccharides.

In one of the embodiments of this method the immobilized polysaccharides are disaccharides A and B - agglutinates of red blood cells, which determine the group of human blood. For the quantitative analysis of the polysaccharide-directed antibody in step *c)*, steps *a)*-*b)* are carried out with the known concentrations of the polysaccharide-directed antibody subject to assay, and a calibration dependence curve is plotted for determining the content of the polysaccharide-directed antibody under analysis in the sample. In different embodiments said method can comprise an immunoassay of any type (direct, indirect, competitive, sandwich-type and others). Detection of the formed polysaccharide-antibody complex in step *b)* is carried out fluorimetrically, chemiluminometrically, mass-spectrometrically or by carrying out the immuno-PCR followed by registration of the PCR results on the same microchip.

The calibration dependence for determining antibodies against polysaccharides can be obtained directly on the microchip, if we use a microchip containing gel cells with immobilized antibodies against the given polysaccharides in different concentrations. The microchip is incubated with the sample containing antibodies against polysaccharides in an unknown concentration and with labeled developing antibodies (sandwich-type immunoassay), and from the gel elements containing the immobilized antibodies against the given polysaccharides in the known concentrations the signal vs. concentration dependence is derived, which is used as the calibration curve for determining the unknown concentration of the antigen.

Yet another aspect of the present invention is a method of multiple parallel immunoassay of polysaccharides on the biological microchip according to the first aspect of the present invention, the microchip containing immobilized polysaccharides or antibodies against polysaccharides, said method contemplating:
*a)* incubation of the biological microchip in a reaction medium comprising a sample containing polysaccharides subject to analysis, for the formation of a polysaccharide-antibody complex, which incubation, if necessary, is carried out under agitation conditions;
*b)* detection of the formed complex;
*c)* quantitative analysis, if necessary, of the polysaccharides.

For the quantitative analysis of the polysaccharide under analysis in step *c)*, steps *a)-b)* are carried out with the known concentrations of the polysaccharide under analysis, a calibration curve is plotted for determining the content of the polysaccharide under analysis in the sample. In different embodiments said method can comprise an immunoassay of any type (direct, indirect, competitive, sandwich-type and others). The detection of the formed polysaccharide-antibody complex in step *b)* is carried out fluorimetrically, chemiluminometrically, mass-spectrometrically, or by carrying out immuno-PCR, followed by registration of the PCR results on the same microchip.

The calibration dependence for determining polysaccharides can be also obtained directly on the microchip, if we use a microchip containing gel elements with immobilized polysaccharides in different concentrations. After the incubation of the microchip with the sample containing polysaccharides subject to assay in an unknown concentration and with labeled indicating antibodies (sandwich-type immunoassay) from the gel elements containing immobilized polysaccharides in the known concentrations the "signal vs. concentration" dependence is obtained, which is used as the calibration curve for determining the unknown concentration of the polysaccharides.

In what follows, the invention will be disclosed in more detail with references to the figures and examples which are given exclusively for the purpose of illustration and explanation of the essence of the claimed invention, but which are not intended for limiting the scope of the claims.

### Brief Description of the Figures

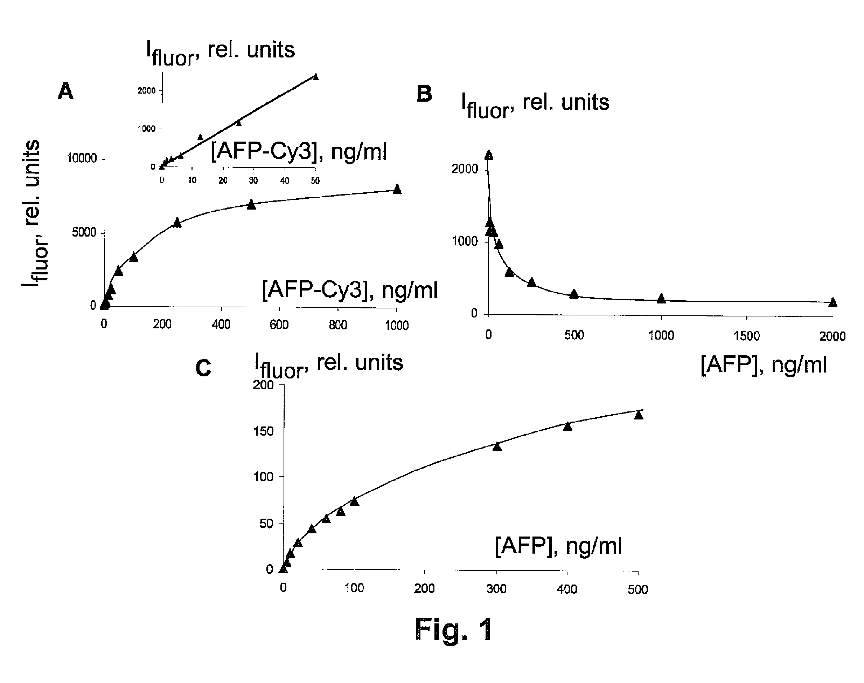

**Figure 1** demonstrates the results of immunoassay of AFP on microchips **A**. Direct immunoassay. The microchips contained gel elements with immobilized antibodies against AFP, 0.1 mg/ml. Dependence of the fluorescence intensity on the concentration of Cy3-labeled AFP in solution. An inset shows fluorescent signals at low AFP concentrations. **B**. Competitive assay. The microchips contained gel elements with immobilized AFP, 0.5 mg/ml. Dependence of the fluorescence intensity on the AFP concentration in solution after incubation of the microchip with solution of Cy3-labeled antibodies against AFP, 0.3 µg/ml. **C**. Sandwich-type immunoassay. The dependence of the fluorescence intensity on the AFP concentration in solution. The microchips contained gel elements with immobilized antibodies against AFP, 0.1 mg/ml; the microchips after incubation with AFP solutions were developed with antibodies to another AFP epitope, labeled with Cy3, 20 µg/ml.
   Each point on the plots is an average of four parallel measurements.

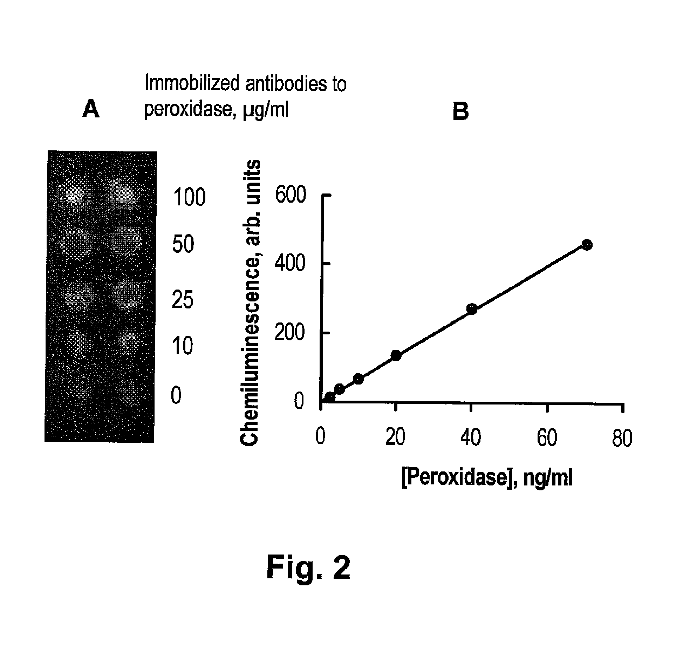

**Figure 2** illustrates chemiluminescent detection of immunoassay results. The microchip contained gel cells with immobilized monoclonal antibodies to horseradish peroxidase in concentration from 10 to 100 µg/ml.
   **A**. Chemiluminescent signals after treating the microchip with solution of peroxidase (10 ng/ml) and with a mixture of substrates, containing luminol, hydrogen peroxide and the enhancer.
   **B**. Dependence of the chemiluminescence intensity on peroxidase concentration in solution applied to the microchip, in the cells of the microchip with the immobilized antibodies in concentration of 100 µg/ml.

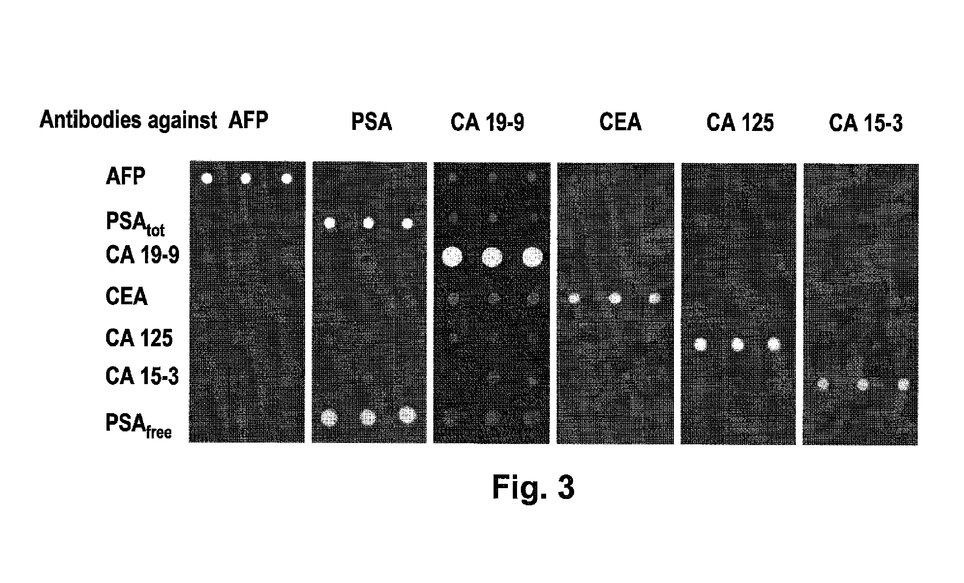

**Figure 3** presents the results of simultaneous sandwich-type immunoassay of several tumor-associated markers on one microchip. Fluorescent image of the microchip containing immobilized antibodies to different oncomarkers after applying the sample and developing with antibodies, labeled with Cy3. Monoclonal antibodies against PSA (free form) are immobilized on the chip, CA 125, CA 19-9, CA 15-3, PSA (total), CEA and AFP (from top to bottom on 3 gel elements). The microchips were treated with blood sera containing (from left to right) PSA, CA 125, CA 19-9, CA 15-3, CEA and AFP and developed with a mixture of Cy3-labeled antibodies against all 6 analyzed oncomarkers.

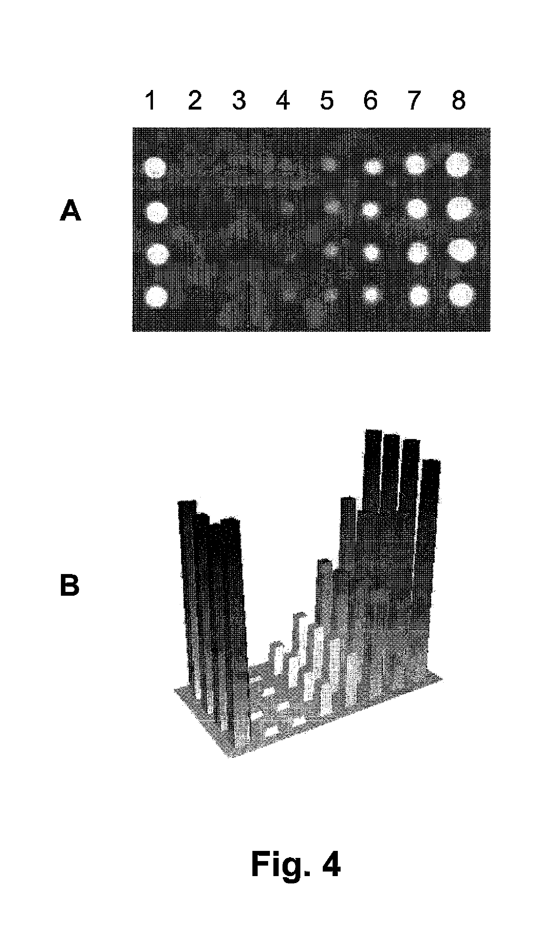

**Figure 4** illustrates sandwich-type immunoassay of prostate-specific antigen (PSA) with the use of a microchip containing gel elements with the immobilized antigen for plotting the calibration curve. **A**. Microchip image obtained after the incubation of the microchip with a solution of PSA, 10 ng/ml, and indicating with Cy3-labeled antibodies. The microchip structure: immobilized antibodies against PSA, 0.1 mg/ml (column 1); empty gels (columns 2 and 3); immobilized PSA for plotting the calibration curve in concentrations corresponding to the working range of the calibration curve (columns 4-8).
   bB. Diagram of the intensity of fluorescent signals of the gel elements of the microchip shown in Fig. **4A**.

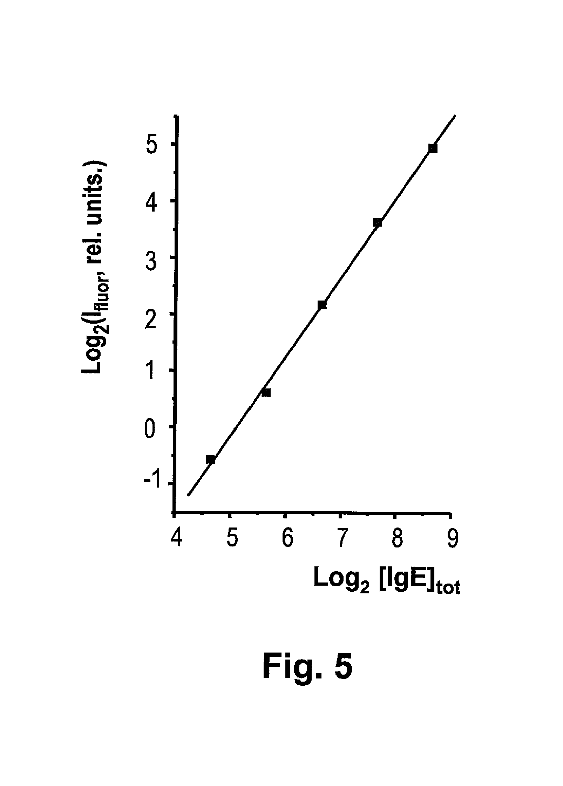

**Figure 5** shows the results of quantitative assay of total immunoglobulin E in blood serum of patients. Dependence of the fluorescent signal intensity on the concentration of total IgE in the blood serum is in logarithmic coordinates.

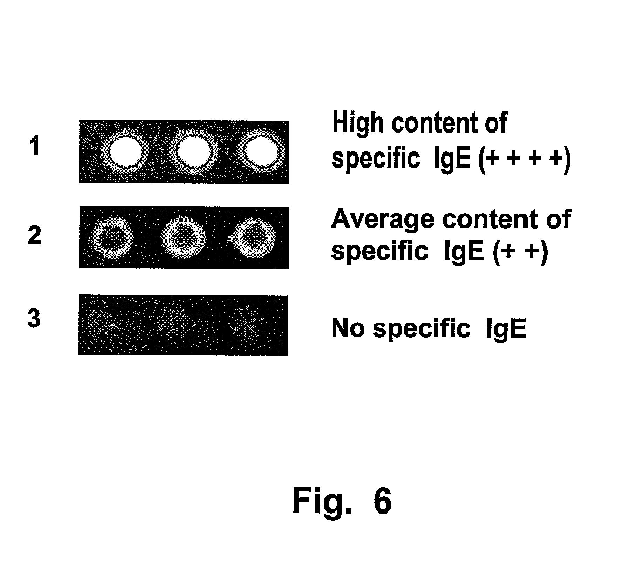

**Figure 6** demonstrates the results of immunoassay of specific immunoglobulin E (allergen of timothy grass) in blood serum of patients. The microchip contained gel elements with the immobilized allergen (timothy grass), 0.5 mg/ml. Fluorescent image of microchips after treating with blood serum and developing with the solution of antibodies against human IgE labeled with Cy3. High content of specific IgE in blood serum (chip 1), average content of specific IgE (chip 2), specific IgE are absent (chip 3).

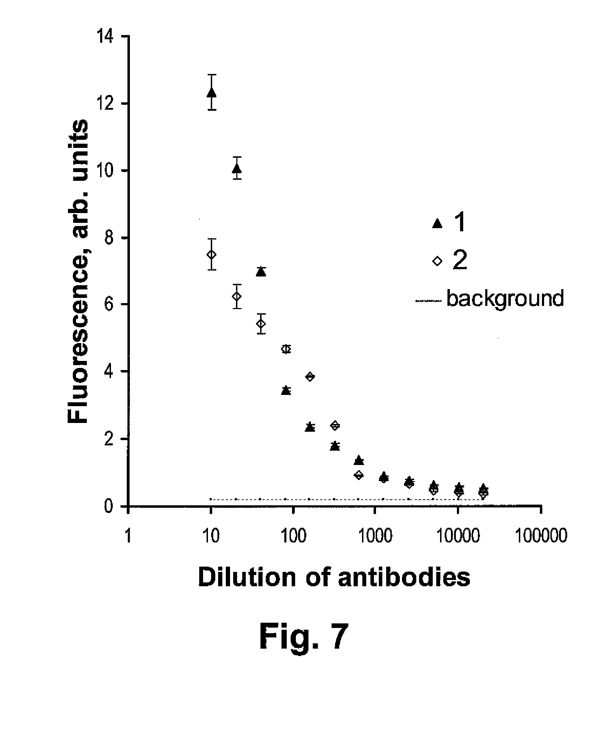

**Figure 7** shows the results of the immunoassay of blood serum, containing antibodies against trisaccharides A (blood group III, curve 2) and trisaccharides B (blood group II, curve 1) with the use of microchips with immobilized trisaccharides A and B.

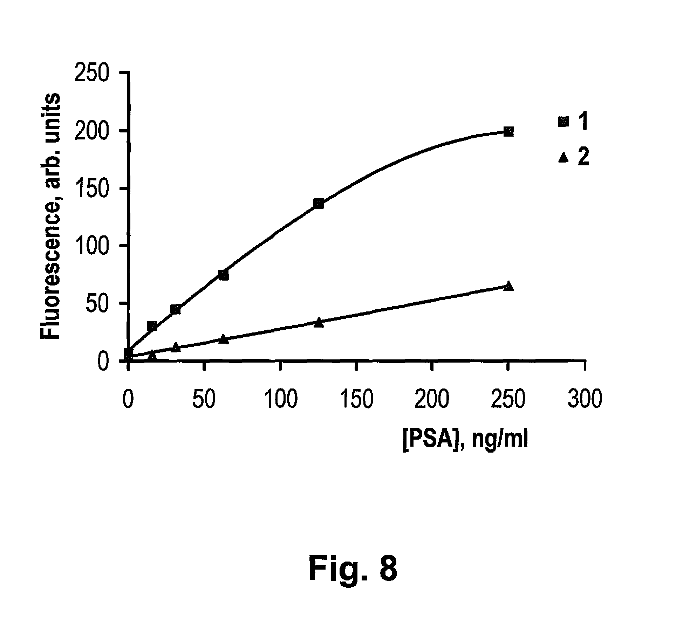

**Figure 8** demonstrates acceleration of immunoassay with the use of sample agitation. Calibration curves for determining prostate-specific antigen (PSA) by the method of sandwich-type immunoassay with the incubation of microchips with the reaction medium, containing PSA, during 2 hours with agitation (1) and without agitation (2). Microchips contained gel elements with immobilized antibodies against PSA, 0.1 mg/ml. The microchips were incubated in the reaction medium containing simultaneously PSA and monoclonal antibodies against another PSA epitope labeled with Cy3, 20 µg/ml.

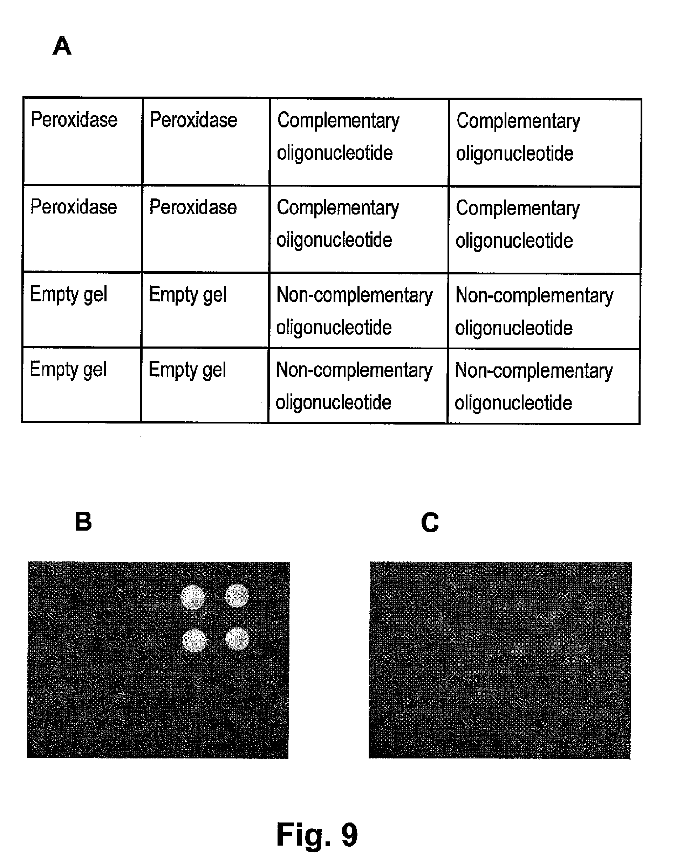

**Figure 9** shows carrying out immunoassay using immuno-PCR method. Structure of microchip **(A)**; fluorescent image of microchip after immuno-PCR with solution containing 1 ng/ml of antibody-oligonucleotide conjugate **(B);** the results of conventional hybridization of the microchip with matrix-complementary oligonucleotide with the same concentration of the conjugate **(C)**.

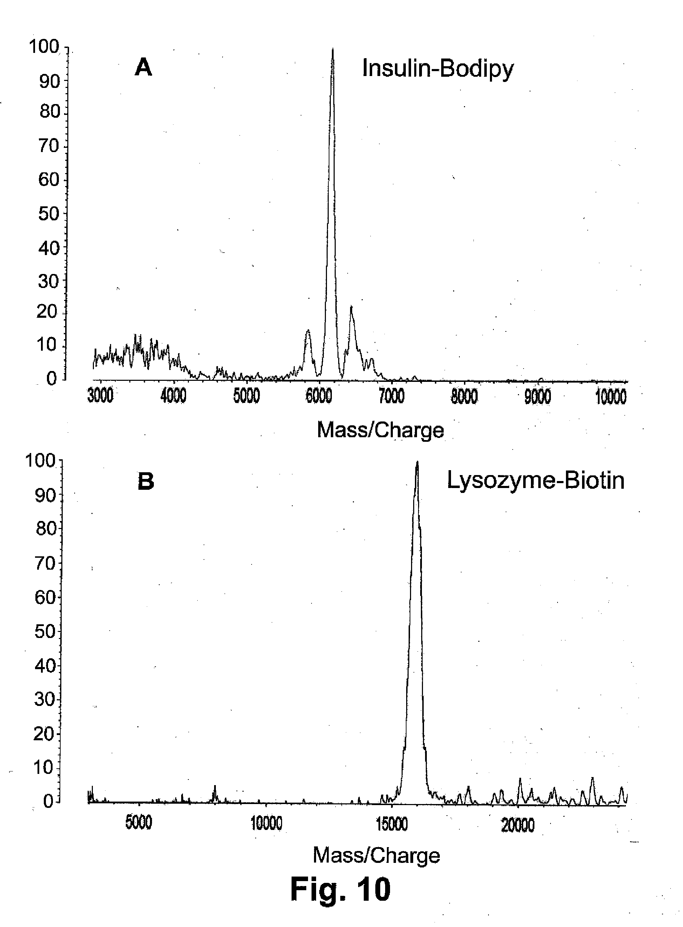

**Figure 10** demonstrates MALDI-TOF mass spectra of insulin, labeled with Bodipy dye **(A)** and of the biotinylated lysozyme **(B),** obtained from the gel cells of microchip with immobilized monoclonal antibodies against insulin and lysozyme, respectively, 0.1 mg/ml, after the incubation of the microchip with solutions of insulin-Bodipy and biotinylated lysozyme in 100 µg/ml concentration.

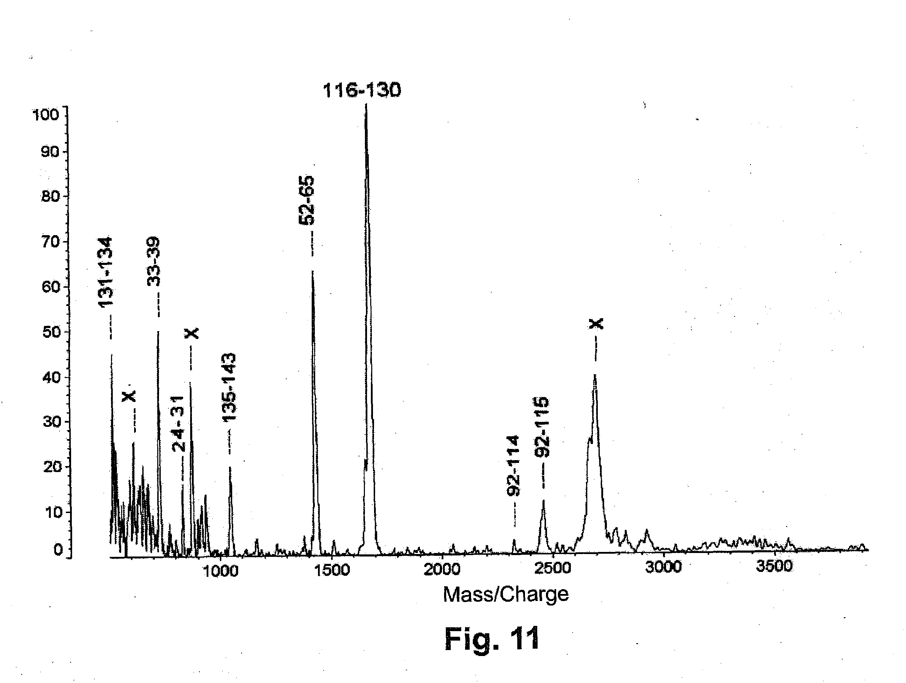

**Figure 11** shows MALDI-TOF mass-spectrum of peptides in the 500 - 3500 m/z region, formed after tryptic hydrolysis of immobilized lysozyme, obtained directly from the gel cell of the microchip. Numerals indicate the number of peptide in the amino acid sequence of the lysozyme; X are peaks obtained in the hydrolysis of trypsin, and the non-identified peaks.

### Detailed Description of the Invention

The present invention provides a biological microchip for multiple parallel immunoassay - an array of hydrogel elements having a preset volume depending on the purpose of the microchip and on the corresponding equipment employed (for example, on the size of the operating unit (pin) of the robot), said array being obtained by the method of photo- or chemically induced radical polymerization. The biological microchip can contain simultaneously biological molecules (ligands) of different nature, in each cell of the microchip an individual compound (ligand) being immobilized. Biological microchips for the immunoassay of compounds are manufactured by the method of polymerization immobilization according to the patented technology (A. D. Mirzabekov, A. Yu. Rubina, S. V. Pan'kov, Composition for polymerising immobilization of biological molecules and method for producing said composition, RF Patent No. 2216547 (B.I.P.M No. 32, 20.11.2003). International Application PCT/RU 01/004204, WO 03/033539), which technology makes it possible to obtain microchips, containing immobilized DNA, proteins and other compounds. Binding of the ligand to the hydrogel matrix is possible directly as the ligand is immobilized in the process of gel formation, and indirectly. Indirect binding of the ligand to the hydrogel matrix can be exemplified by immobilization via formation of specific complexes of avidin (streptavidin)-biotin type such as, formation of a link between avidin immobilized on a hydrogel matrix and a biotinylated antibody, or immobilization via formation of specific complexes of metal - chelator type, for example, formation of a link between nitrilotriacetic acid immobilized on a hydrogel matrix and a nickel atom bound thereto by coordination bonds and recombinant protein containing a polyhistidine fragment, etc.

Compounds of protein and non-protein nature, such as immunoglobulins of any type (IgG, IgM, IgA, IgD, IgE), monoclonal antibodies, polyclonal antibodies, recombinant antibodies, different types of mini-antibodies, including Fab-fragments and single-chain antibodies (scFv and others), aptamers, antigens of different nature (proteins, glycoproteins, polysaccharides etc.), low-molecular substances can act as ligands immobilized in gel cells. The type of ligands utilized in the assay depends on a number of factors, for example, on the affinity of antibodies for a given antigen, on the stability, on the possibility of immobilizing antibodies and introducing a label thereinto (a fluorescent label, an enzyme label by obtaining conjugates, etc.), and also on the method used for carrying out the assay (direct, competitive, sandwich-type immunoassay and others).

The biological microchip allows sequential carrying out different types of reactions. For example, the first reaction can be the interaction of an antigen with an antibody to form a dual antigen-antibody complex or a triple antibody-antigen-antibody complex for antigens of protein nature (immunological reaction). Then, if the antibody or antigen contains an enzyme label, an enzymatic reaction is carried out, for example, oxidation of luminol with hydrogen peroxide in the presence of enhancers, catalyzed by horseradish peroxidase, as a result of which emission of light (chemiluminescence) takes place. In the case the antibody or antigen is a conjugate with an oligonucleotide, after carrying out immunological reaction, polymerase chain reaction (PCR) can be carried out directly on the microchip.

The next reaction is the formation of a specific complex between the oligonucleotides obtained as a result of the PCR, with complementary oligonucleotides immobilized in other cells of the same microchip.

As objects subject to quantitative immunoassay by the method of the invention there are preferably proposed:
tumor-associated antigens, which are markers of oncological diseases, for example: alpha-fetoprotein (AFP) - marker of carcinoma of the liver; general and free prostate-specific antigen (PSA), - marker of prostate cancer; carcinoembryonic antigen (CEA) - marker of cancer of the gastrointestinal tract; cancer antigen 125 (CA 125) - marker of ovarian cancer, of hysterocarcinoma; cancer antigen 19-9 (CA 19-9) 015 - marker of cancer of the pancreas; cancer antigen 15-3 (CA 15-3)- marker of cancer of the mammary gland; total human immunoglobulin E; specific antibodies against allergens;
antibodies against polysaccharides, in particular, against trisaccharides A and C - agglutinogens of red blood cells, determining the group of human blood; polysaccharides.

The immunoassay of tumor-associated antigens is carried out with the use of biological microchips containing immobilized antibodies against said tumor-associated antigens or immobilized tumor-associated antigens. In the case of immunological determination of total human immunoglobulin E, as the immobilized ligands of the biological microchip antibodies against human immunoglobulins E or human immunoglobulins E are used. For the immunoassay of specific antibodies against different allergens, the allergens are immobilized ligands of the biological microchip. The immunoassay of specific antibodies to polysaccharides and of polysaccharides is carried out with the use of the biological microchip with immobilized polysaccharides and antibodies against polysaccharides.

A general procedure for carrying out immunoassay with the use of biological microchips is described in Example 1. For the immunoassay of tumor-associated antigens - markers of oncological diseases, antibodies to said tumor-associated antigens or tumor-associated antigens were used as the immobilized ligands of the biological microchip (Examples 2, 4, 5). In Example 6 a biological microchip is described for the immunoassay of total human immunoglobulin E with the use of antibodies against immunoglobulins E as the immobilized ligands. In Example 7 a biological microchip is described for the immunoassay of specific antibodies against the timothy grass allergen with the use of the immobilized (timothy grass) allergens. For the immunoassay of specific antibodies to polysaccharides (human blood group determination) a biological microchip with immobilized trisaccharides A and B was used (Example 8).

The present invention also provides a method of carrying out immunoassay on a biological microchip, said method contemplating:
*a)* incubation of a biological microchip in a reaction medium comprising a sample, containing compounds under analysis for the formation of immune complexes with ligands immobilized on the microchip, which incubation, if necessary, is carried out under agitation conditions;
*b)* detection of the formed complex;
*c)* if necessary, quantitative analysis of the compound under analysis.

As the reaction medium in carrying out an assay, buffer solutions are used, which are commonly used in immunoassay, for example, a phosphate buffer, tris-HCl and others For decreasing nonspecific interactions, the reaction medium is added with polyvinyl alcohol, bovine serum albumin, saccharose, dry milk proteins, and the like (so-called "blocking solutions", well known to those skilled in the art). When carrying out sandwich-type immunoassay, a variant is possible, when the reaction medium in step a) additionally contains the compound under analysis and developing antibodies. In this case the time of assay is shortened, since the incubation of the microchip with the compound under analysis and the indication occur simultaneously.

The registration of the results of immunoassay is conducted in terms of the intensity of fluorescence, chemi- or bioluminescence, with the use of mass spectrometry directly from the gel cells of the microchip, and also by carrying out the immuno-PCR, followed by registration of the PCR results on the same microchip. In the fluorescent registration of the results, antigens and antibodies are used, labeled with fluorescent dyes, for example with Texas red, with fluorescein, with cyanine 3 and 5 (Cy3, Cy5), but without being limited thereto. The registration of fluorescent signals from the cells of the microchip is carried out with the aid of fluorescent microscopes or scanning microscopes of different types, making it possible to record the signal from the employed fluorescent label (Examples 2, 4-9). When registration of the results is carried out by the intensity of chemi- or bioluminescence, conjugates of antibodies or avidin with horseradish peroxidase, with alkaline phosphatase, with β-galactosidase, with luciferase or with other enzymes are used, giving chemi- or bioluminescent signals as a result of enzymatic reactions, for example, peroxidase-catalyzed oxidation of luminol with hydrogen peroxide in the presence of an enhancer (Example 3). Chemi- or bioluminescent signals are recorded with the aid of CCD- cameras or other recording devices.

In the case of using mass spectrometry, after the interaction of the biological microchip with the sample and formation of the complex "immobilized ligand - compound under analysis", the identification of the compound under analysis is carried out directly on the hydrogel element of the microchip, using the results of the direct mass-spectral assay of the compound bound to the ligand.

The procedure of registering the results of assay in this case is as follows: after washing the microchip free from the non-specifically sorbed proteins, elution is carried out under the conditions destroying the complex formed by the compound under analysis and the immobilized ligand, direct mass-spectral assay is carried out from each gel element, and the compound is identified by its molecular weight and ability to bind to the specific ligand (Example 11).

Identification of the compound of protein nature, bound to the immobilized ligand, is carried out also after proteolytic hydrolysis of protein complexes in the gel cells. For this purpose the microchip is treated with solution of a protease (trypsin, chymotrypsin, pepsin, papain, subtilisin, proteinase K, Asp-N-endopeptidase and others, but not limited thereto), and further each element of the microchip is analyzed with the aid of mass spectrometry. If the ligand immobilized in the gel is of non-protein nature, then the obtained mixture of peptides corresponds to one protein which has formed a complex with the given ligand. The molecular weights of peptides, obtained as a result of mass-spectrometric assay (peptide map) are compared with the peptide maps of the known proteins from the database, and the unknown protein is identified.

If the ligand immobilized in the gel is of protein nature, then this ligand itself undergoes the effect of protease. Therefore concurrently a control experiment is carried out on proteolytic splitting of the protein immobilized in the gel cell, followed by mass-spectrometric assay, and the molecular weights of the peptides formed as a result of hydrolysis of the immobilized protein are determined. After the proteolytic hydrolysis of the "protein ligand-unknown protein" complex and mass-spectrometric assay a set of peptides corresponding to a mixture of two proteins is obtained. The set of peptides formed after the hydrolysis of the unknown protein is found by subtraction, and the unknown protein is identified with the aid of the database, using the peptide maps of the known proteins (Example 12). One more method of detecting the results of immunoassay on biological microchips contemplates carrying out immuno-CPR reaction and registering the results of this reaction on the same microchip. In this case the biological microchip simultaneously contains in different gel elements immobilized ligands (antibodies or antigens) and immobilized oligonucleotides (Example 10). For instance, the biological microchip contains immobilized antibodies against the compound under analysis. The sample under investigation is applied to the microchip, specific binding antigen-antibody occurs, and the conjugate of the antibody with the oligonucleotide is used as the developing antibodies (a variant of sandwich-type immunoassay). The next step is carrying out PCR: to the same microchip a mixture of reagents for PCR is applied and the required number of reaction cycles is carried out; then the conditions (temperature etc.) are changed in such a manner that the resultant fluorescently-labeled PCR-product forms a specific complex with complementary oligonucleotides immobilized in other cells of the microchip. The microchip cells containing immobilized antibodies and immobilized non-complementary oligonucleotides serve as negative control. After washing the microchip, the fluorescent signal is registered.

The use of this method involves at least a thousandfold increase of the signal due to the fact that in the course of PCR a several orders of magnitude larger amount of a fluorescently labeled oligonucleotide, which is complementary to the oligonucleotide in the conjugate with the antibody, is formed, and this amount of the fluorescently labeled product determines the final value of the registered signal (Example 10).

The immunoassay carried out on hydrogel microchips can be realized as an assay of any type: direct, indirect, competitive, sandwich-type variant and others (Example 1).

For direct immunoassay the biological microchip contains immobilized antibodies against the compound under analysis, and the reaction medium in step *a)* contains the compound under analysis which forms a specific immune complex with the immobilized antibodies. The signal registered from the corresponding gel cells of the microchip is proportional to the concentration of the compound under analysis.

The biological microchip for the competitive immunoassay contains immobilized antibodies or immobilized antigens. In the first case to the reaction medium in step *a)* is further a labeled compound under analysis, and there takes place a competition between the specially added labeled compound and the unlabeled compound being present in the sample under assay for binding to the immobilized antibodies. In the variant of competitive assay with the use of a microchip with immobilized antigens to the reaction medium in step *a)* is further added labeled antibodies against the compound under analysis, and there takes place a competition of the labeled antibodies for binding to the compound under analysis in solution and the compound immobilized on the microchip. In the case of competitive immunoassay the higher is the signal registered from the corresponding gel cells of the microchip, the smaller is the concentration of the compound under analysis in the sample.

The biological microchip for sandwich-type immunoassay contains immobilized antibodies against the compound under analysis, and the reaction medium in step *a)* contains the compound under analysis. After the formation of the specific complex "antigen - immobilized antibody" the microchip are developed with labeled antibodies specif to another epitope of the compound under analysis. The signal recorded from the corresponding gel cells of the microchip is proportional to the concentration of the compound under analysis. For the sandwich-type immunoassay a variant is also possible, when the reaction medium in step *a)* besides the compound under analysis, additionally contains developing antibodies, and thereby the assay time becomes reduced, since the incubation of the microchip with the compound under analysis and the development occur simultaneously.

Direct and competitive analyses on the microchips can be carried out for both low-molecular compounds and for compounds of protein nature. The sandwich variant of the immunoassay is possible only for compounds of protein nature, for which there exist antibodies specific to different epitopes of the protein molecule: a binding antibody and a developing antibody.

The quantitative detection of compounds on the biological microchip for all variants of assay is effected by carrying out steps *a)-b)* with the known concentrations of the compound under analysis in standard (reference) solution and plotting the calibration dependence curve "signal vs. the concentration of the compound under analysis", which serves for determining the unknown concentration of the compound under analysis in the sample.

The calibration curve for the quantitative determination of the antigen can be also obtained with the use of a microchip containing immobilized ligands. For example, the microchip contains gel elements with immobilized antibodies against the antigen under analysis and gel elements with the immobilized antigen in different concentrations. After carrying out sandwich-type immunoassay, i.e., after the incubation of the microchip with the sample and labeled developing antibodies, the signals obtained from the gel elements with the known concentrations of the immobilized antigen serve for plotting the calibration curve, using which the unknown concentration of the antigen in the sample is determined.

The detection limit of the compound under analysis is determined as the concentration corresponding to the intensity of the fluorescent/chemiluminescent signal, 3 times exceeding the spread of the background signal.

The proposed method of quantitative immunoassay with the use of biological microchips is noted for high sensitivity (Table 1) and reproducibility (coefficient of variation 10% (Example 2)).

The use of microchips makes it possible to carry out multiple, quantitative, parallel immunoassay of samples for many parameters, whereby the efficiency of the assay is sharply increased and the amount of material required for the investigation is considerably reduced: 20 µl of a sample to be investigated is sufficient for assay. The biological microchip for parallel multiple immunoassay contains immobilized antibodies against several compounds and/or several immobilized antigens. In the case of direct immunoassay the reaction medium contains sample comprising one or several compounds under analysis, and the signals of the cells of the microchip are registered after the incubation of the microchip with the sample containing the corresponding immobilized antibodies. For the competitive assay the reaction medium in step *a)* additionally contains a mixture of labeled antibodies to all compounds under analysis or a mixture of all labeled compounds under analysis. In the case of sandwich-type immunoassay the development of the microchip after incubation in the reaction medium containing the sample is carried out with a mixture of labeled antibodies against all compounds under analysis. Example 5 demonstrates simultaneous parallel assay of several antigens, for example, tumor-associated markers, on one microchip without loss of the method sensitivity.

If necessary, the step of the incubation of the microchip in the reaction medium, comprising a sample containing compounds under analysis (step *a)* is carried out under agitation conditions which considerably decrease the assay time due to the acceleration of diffusion, shifting the dynamic equilibrium toward formation of a specific antigen-antibody complex (Example 9). Agitating can be accomplished, for instance, by changing the direction of the flow of the reaction solution from direct to reverse with the aid of pumps of different types, by ultrasound, by electrophoresis, the choice being not limited to the indicated techniques.

The present invention provides a method of multiple parallel immunoassay of oncomarkers on a biological microchip comprising immobilized antibodies against oncomarkers and/or immobilized oncomarkers. During the first step of the immunoassay (step *a)*) incubation of the biological microchip is carried out in the reaction medium comprising a sample containing oncomarkers under analysis, for forming an immune complex with ligands immobilized on the microchip, which incubation, if necessary is carried out under agitation conditions. The detection of the formed complex (step *b)*) is carried out fluorimetrically, chemiluminometrically, mass-spectrometrically or by carrying out immuno-PCR with subsequent registration of the PCR results on the same microchip. For the quantitative determination (step *c)*), steps *a) - b)* are carried out with the known concentrations of the oncomarker under analysis, and a calibration dependence curve is plotted, using which the content of the oncomarker under analysis in the sample is determined.

The assay of tumor-associated markers on microchips can be performed in the form of immunoassay of any type (direct, indirect, competitive, sandwich-type, and others). Direct assay of oncomarkers is carried out with the use of biological microchips with immobilized antibodies against oncomarkers. For instance, direct assay of alpha-fetoprotein (AFP) (Example 2) was carried out with the use of microchips with immobilized antibodies against AFP, and AFP contained fluorescent label Cy3. The registered signal from the cells of the microchip with the immobilized antibodies was proportional to the concentration of the antigen (Fig. 1A).

In carrying out competitive assay microchips with immobilized antibodies against oncomarkers or with immobilized oncomarkers are used. In the first variant the reaction medium in step *a)* additionally contains a specially added labeled oncomarker, and a competition takes place between the labeled and unlabeled oncomarker for binding to the immobilized antibodies. In the case of competitive assay with the use of biological microchips with immobilized oncomarkers, the reaction medium in step *a)* additionally contains specially added labeled antibodies against the oncomarker under analysis, and there takes place a competition of the labeled antibodies for binding to the compound in solution and the compound (oncomarker) immobilized on the microchip. For instance, in the competitive assay of AFP, described in Example 2, the microchip contained gel elements with the immobilized AFP, and the reaction medium in step *a)* additionally contained Cy3-labeled antibodies to AFP. For the competitive assay the registered signal of the microchip cells is the higher the smaller is the concentration of the compound under analysis in the sample (Fig. 1B).

In the case of sandwich-type immunoassay the microchip contains immobilized antibodies against the oncomarkers under analysis, and the reaction medium in step *a)* contains the compound under analysis. After the formation of the "antigen-antibody" complex the microchip is developed with labeled antibodies specific to another epitope of this oncomarker. In Example 2 there is described sandwich-type immunoassay of AFP on a microchip with immobilized antibodies against AFP with the development with second antibodies against AFP, containing fluorescent label Cy3. The registered signal of the cells of the microchip with the immobilized antibodies is proportional to the AFP concentration (Fig. 1C). For the sandwich-type immunoassay it is also possible that the reaction medium in step *a)* simultaneously with the compound under analysis additionally contains developing antibodies, and, thus, the incubation of the microchip with the compound under analysis and the development occur simultaneously. In Example 9 the immunoassay of PSA with the incubation of microchips was carried out in the reaction medium simultaneously containing PSA and Cy3-labeled monoclonal antibodies against another PSA epitope.

A variant is also possible, when the microchip simultaneously with immobilized antibodies against oncomarkers contains gel elements with immobilized oncomarkers in different concentrations for plotting a calibration dependence curve. In this case the incubation of the microchip with a sample containing an oncomarker in unknown concentration, and with labeled antibodies against the oncomarker under analysis (sandwich-type immunoassay), and from the gel elements with the known concentration of the immobilized oncomarker the dependence of "signal vs. oncomarker concentration" is obtained. This dependence (calibration curve) is used for determining the unknown concentration of the oncomarker in the sample. An image of the microchip for the sandwich-type immunoassay of the prostate-specific antigen (PSA) with the use of a microchip comprising gel elements with the immobilized antigen for plotting the calibration curve is presented in Fig. 4. The microchip contained immobilized antibodies against PSA and immobilized PSA for plotting the calibration curve in concentrations corresponding to the working range of the calibration curve.

The present invention also provides a method of multiple parallel immunoassay of total human immunoglobulin E on a biological microchip containing immobilized antibodies against human immunoglobulins E (anti-IgE) and/or immobilized human immunoglobulins E (IgE). In the first step (step *a)*) incubation of the biological microchip in the reaction medium comprising a sample containing immunoglobulins E is carried out for forming an immune complex with the ligands immobilized on the microchip, which incubation, if necessary, is carried out under agitation conditions. The detection of the formed complex (step *b)*) is carried out fluorimetrically, chemiluminometrically, mass-spectrometrically or by carrying out immuno-PCR with subsequent registration of the PCR results on the same microchip. For the quantitative determination (step *c))* steps *a)* - *b)* are carried out with the known concentrations of the immunoglobulin E under analysis and the calibration dependence is plotted, using which the content of the immunoglobulin E under analysis in the sample (human blood serum) is determined.

The assay of human immunoglobulin E on microchips can be carried out in the form of immunoassay of any type (direct, indirect, competitive, sandwich-type and others). Microchips with immobilized antibodies against immunoglobulin E are used for the direct assay. The reaction medium contains labeled immunoglobulin E in the case of fluorescent or chemiluminescent detection or unlabeled immunoglobulin E in the case of mass-spectral detection.

In carrying out competitive assay microchips with immobilized immunoglobulin E or with immobilized antibodies against immunoglobulin E. are used. In the first variant the reaction medium in step *a)* additionally contains specially added labeled immunoglobulin E, and a competition between the labeled and unlabeled immunoglobulin E for binding to the immobilized antibodies occurs. In another variant (microchip with immobilized immunoglobulin E) the reaction medium in step *a)* additionally contains specially added labeled antibodies against immunoglobulin E, and a competition of the labeled antibodies for binding to the immunoglobulin E in solution and the immunoglobulin E immobilized on the microchip takes place.

The biological microchip for the sandwich-type immunoassay of human immunoglobulin E contains immobilized antibodies against the immunoglobulin E (anti-IgE), and reaction medium in step *a)* contains the human immunoglobulin E subject to analysis (blood serum). After the formation of the "antigen-antibody" complex the microchip is developed with second labeled antibodies against human immunoglobulin E. The sandwich-type immunoassay of IgE on the microchip with immobilized anti-IgE with the development with fluorescently labeled anti-IgE is described in Example 6. The registered signal of the microchip cells with the immobilized anti-IgE is proportional to the concentration of IgE in solution or in human blood serum (Fig. 5). For the sandwich-type immunoassay of immunoglobulin E a variant is possible, when the reaction medium in step *a)* besides the compound under analysis additionally contains other labeled anti-IgE, and, thus, the incubation of the microchip with the compound under analysis and the development occur simultaneously.

The calibration dependence for determining the total immunoglobulin E can be also obtained directly on the microchip, if we use a microchip containing gel elements with the immobilized IgE in different concentrations. The microchip is incubated with a sample (blood serum) containing IgE in an unknown concentration, and with second labeled antibodies against IgE (sandwich-type immunoassay), and from the gel elements with the immobilized IgE the dependence of signal on the concentration of IgE is obtained, which is used as the calibration curve for determining the unknown concentration of IgE in the blood serum.

The present invention also provides a method of multiple parallel immunoassay of allergen-specific immunoglobulins E on a biological microchip containing immobilized specific allergens. The first step of the assay (step *a))* is the incubation of the biological microchip in the reaction medium comprising a sample containing allergen-specific immunoglobulins E, for forming an immune complex with the allergens immobilized on the microchip, which incubation, if necessary, is carried out under agitation conditions. The detection of the formed complex (step *b))* is effected fluorimetrically, chemiluminometrically, mass-spectrometrically or by carrying out the immuno-PCR with the subsequent registration of the PCR results on the same microchip. For the quantitative determination (step *c),* steps *a)-b)* are carried out with the known concentrations of the allergen-specific immunoglobulin E subject to analysis, and a calibration dependence is plotted, using which the content of the allergen-specific immunoglobulin E under analysis in the sample (human blood serum) is determined.

An assay of allergen-specific immunoglobulin E on microchips can be carried out in the form of immunoassay of any type (direct, indirect, competitive, sandwich-type and others). Microchips with immobilized antibodies against immunoglobulin E are used for the direct assay. The reaction medium contains labeled immunoglobulin E in the case of fluorescent or chemiluminescent detection or unlabeled immunoglobulin E in the case of mass-spectral detection.

When carrying out competitive assay on microchips with immobilized allergens, the reaction medium in step *a)* additionally contains specially added labeled antibodies against human immunoglobulins E, and a competition takes place between the labeled and unlabeled antibodies for binding to the allergens immobilized on the microchip.

When carrying out sandwich-type immunoassay of allergen-specific immunoglobulins E, the reaction medium in step *a)* additionally contains allergen-specific human immunoglobulin E. After the formation of the "immobilized allergen-antibody" complex, the microchip is developed with labeled antibodies against human immunoglobulins E. In this case it is possible to use the same developing antibodies as for the assay of total human immunoglobulin E, and, correspondingly, it is possible to manufacture a microchip for a simultaneous assay of total and allergen-specific immunoglobulins in human blood serum. The sandwich-type immunoassay of a specific IgE (timothy grass allergen) in blood serum when the microchip is developed with fluorescently labeled anti-IgE is described in Example 7. For the sandwich-type immunoassay of specific IgE a variant is also possible, when the reaction medium in step *a)* besides the compound subject to analysis additionally contains labeled antibodies against human immunoglobulins E, and, thus, incubation of the microchip with the compound under analysis and the development take place simultaneously.

For the quantitative assay of the allergen-specific IgE a microchip is also used, which simultaneously with immobilized allergens contains gel elements with immobilized allergen-specific IgE in different concentrations for obtaining the calibration dependence on the same microchip. The microchip is incubated with the sample containing the allergen-specific IgE in an unknown concentration and labeled antibodies against human immunoglobulins (sandwich-type immunoassay), and from the gel elements with the immobilized allergen-specific IgE in the known concentrations the dependence of signal on the concentration of the antigen under analysis is obtained, which is used for determining the unknown concentration of the allergen-specific IgE in the sample.

The present invention also provides a method of the multiple parallel immunoassay of antibodies against polysaccharides on a biological microchip containing immobilized polysaccharides or antibodies against polysaccharides. In the first step (step *a)* incubation of the biological microchip is carried out in a reaction medium comprising a sample containing antibodies against polysaccharides subject to analysis, for forming an immune "polysaccharide-antibody" complex, which incubation, if necessary, is carried out under agitation conditions. Detection of the formed complex (step *b*) is effected fluorimetrically, chemiluminometrically, mass-spectrometrically or by carrying out immuno-PCR followed by registration of the PCR results on the same microchip. For the quantitative determination (step *c)*, steps *a)-b)* with the known concentrations of the antibodies under analysis against polysaccharides are carried out, and a calibration dependence is plotted, using which the content of the compound being analyzed in the sample is determined.

An assay of antibodies against polysaccharides on microchips can be effected as an immunoassay of any type (direct, indirect, competitive, sandwich-type immunoassay, and others). For direct assay microchips with immobilized polysaccharides are used. The reaction medium in step *a)* contains labeled antibodies subject to analysis against polysaccharides in the case of fluorescent or chemiluminescent detection or unlabeled antibodies against polysaccharides in the case of mass-spectral detection.

When carrying out competitive assay of antibodies against polysaccharides, there can be used both microchips with immobilized antibodies against the polysaccharides and with immobilized polysaccharides. In the first variant the reaction medium in step *a)* additionally contains a specially added labeled polysaccharide, and there takes place a competition between the antibodies in solution and the antibodies immobilized on the microchip for binding to the labeled polysaccharide. For the competitive assay of antibodies against polysaccharides with the use of biological microchips with immobilized polysaccharides, the reaction medium in step *a)* additionally contains specially added labeled antibodies against the polysaccharide under analysis and there takes place a competition between the labeled and unlabeled antibodies for binding to the polysaccharide immobilized on the microchip.

For the sandwich-type immunoassay of antibodies against polysaccharides microchips with immobilized polysaccharides are used. The reaction medium in step *a)* contains antibodies under analysis, and there takes place formation of a "polysaccharide-antibody" complex. The microchip is further developed with second antibodies containing a fluorescent label. For instance, an assay of monoclonal antibodies against trisaccharides A and B - agglutinogens of red blood cells, which determine the group of human blood was effected on microchips with immobilized trisaccharides A and B (Example 8). After the incubation of the microchip with human blood serum, the microchip was developed with murine antibodies IgM against human antibodies, labeled with Cy5.

For the sandwich-type immunoassay of antibodies against polysaccharides, it is also possible that the reaction medium in step *a)* besides the compound under analysis additionally contains labeled antispecific antibodies, and, thus, the incubation of the microchip with the compound under analysis and the development occur simultaneously.

The calibration dependence for determining the antibodies against polysaccharides can be obtained directly on the microchip, if we use a microchip containing gel cells with immobilized antibodies against the given polysaccharides in different concentrations. The microchip is incubated with the sample containing antibodies against polysaccharides in an unknown concentration, and with labeled developing antibodies (sandwich-type immunoassay), and from the gel elements containing the immobilized antibodies against the given polysaccharides in the known concentrations the dependence of signal on the concentration is obtained, which is used as the calibration curve for determining the unknown concentration of antibodies against polysaccharides.

The present invention also provides a method of multiple parallel immunoassay of polysaccharides on a biological microchip containing immobilized polysaccharides or antibodies against polysaccharides. In the first step (step *a))* the biological microchip is incubated in the reaction medium comprising a sample containing polysaccharides subject to analysis, for forming an immune "polysaccharide-antibody" complex, which incubation, if necessary is carried out under agitation conditions. Detection of the formed complex (step *b*} is carried out fluorimetrically, chemiluminometrically, mass-spectrometrically or by carrying out immuno-PCR followed by registration of the PCR results on the same microchip. For the quantitative determination (step *c)*, steps *a)-b)* are carried out with the known concentrations of the polysaccharides subject to analysis, and a calibration dependence is plotted, using which the content of the subject to analysis in the sample is determined.

An assay of polysaccharides on microchips can be effected as an immunoassay of any type (direct, indirect, competitive, sandwich-type immunoassay, and others). For direct assay microchips with immobilized antibodies against polysaccharides are used. The reaction medium in step *a)* contains labeled polysaccharides subject to analysis in the case of fluorescent or chemiluminescent detection or unlabeled polysaccharides in the case of mass-spectral detection.

When carrying out competitive assay of polysaccharides, as well as the assay of antibodies against polysaccharides, both microchips with immobilized antibodies against the polysaccharides and with immobilized polysaccharides can be used. In the first variant the reaction medium in step *a)* additionally contains a specially added labeled polysaccharide, and there takes place a competition between the labeled and unlabeled polysaccharide for binding to the immobilized antibodies. In another variant (microchip with immobilized polysaccharides) the reaction medium in step *a)* additionally contains specially added labeled antibodies against the polysaccharide subject to analysis, and there takes place a competition of the labeled antibodies for binding to the polysaccharide in solution and the polysaccharide immobilized on the microchip.

The biological microchip for the sandwich-type immunoassay of polysaccharides contains immobilized antibodies against the polysaccharides under assay, and the reaction medium in step *a)* contains the polysaccharide under analysis. After the formation of the "polysaccharide-antibody" complex, the microchip is developed with second labeled antibodies against this polysaccharide. For the sandwich-type immunoassay a variant is also possible, when the reaction medium in step *a)* besides the polysaccharides subject to analysis additionally contains second labeled antibodies against the analyzed polysaccharides, and, thus, the incubation of the microchip with the compound under analysis and the development occur simultaneously.

The calibration dependence for determining polysaccharides also can be obtained directly on the microchip, if we use a microchip, containing gel cells with the immobilized polysaccharides in the different concentrations. The microchip is incubated with the sample containing polysaccharides under analysis in an unknown concentration, and with the labeled developing antibodies (sandwich-type immunoassay), and from the gel elements, containing the immobilized polysaccharides in the known concentrations, the dependence of signal on the concentration is obtained, which is used as the calibration curve for detennining the unknown concentration of polysaccharides.

The Examples given below are intended not for limiting the claims, but exclusively for illustrating separate embodiment of the present invention.

### Example 1. Quantitative immunoassay with the use of hydrogel microchips

For quantitative immunoassay hydrogel microchips were prepared, containing elements with immobilized proteins (antibodies and/or antigens). For decreasing nonspecific interactions in all variants of the assay the microchips were preliminarily incubated in a 0.01 M phosphate buffer, pH 7.2, containing 0.15 M NaCl, 1% of polyvinyl alcohol (PVA-50) or in the same buffer, containing 3% of bovine serum albumin (BSA) and 4% of saccharose ("blocking buffer"), 2 hrs. at room temperature. Before carrying out the assay, solutions of an antigen (sample) were diluted with 0.01 M phosphate buffer, pH 7.2, by containing 0.15 M NaCl, 0.15% of PVA-50 and 0,15% of polyvinylpyrrolidone 360.

Direct immunoassay. Solutions of the fluorescently labeled antigen (for instance, of an oncomarker), 20 µl, were applied on microchips containing gel elements with immobilized monoclonal antibodies to the antigen under analysis and antibodies to other antigens for control of cross-interactions, and microchips were maintained at room temperature for 1 h. After washing the microchips in 0.01 M phosphate buffer, pH 7.2, 0.15 M NaCl, 0.1% Tween-20 (15 min, room temperature), the intensity of the fluorescent signals of the gel elements of microchips was measured.

For competitive immunoassay a microchip with an immobilized antigen was used. The microchip containing the antigen under analysis was incubated with a solution of fluorescently labeled monoclonal antibodies (0.3 µg/ml) or of conjugates of antibodies with peroxidase for 2 h. The mixture (20 µl) after the incubation was applied to the microchip and maintained for 2 hrs. at 37°C. After washing in 0.01 M phosphate buffer, pH 7.2, 0.15 M NaCl, 0.1 % Tween-20 (15 min, room temperature), the intensity of fluorescent or chemiluminescent signals was measured.

In the sandwich-type variant of immunoassay, the antigen solution, 20 µl, was added to the antibodies immobilized on the microchip, and the microchips were incubated overnight at room temperature. After a short-term (15 min) washing in 0.01 M phosphate buffer, pH 7.2, 0.15 M NaCl, 0.1% Tween-20, microchips were developed with the fluorescent- labeled antibodies (or the conjugates of antibodies with peroxidase), specific to another epitope of the antigen (40 min, room temperature). After washing (0.01 M phosphate buffer, pH 7.2, containing 0.15 M NaCl, 0.1% Tween-20, 15 min, room temperature) the intensity of fluorescent or chemiluminescent signals was measured.

For the sandwich-type immunoassay a variant was also used, when microchips were incubated at room temperature in the reaction medium containing an antigen in a 0.01 M phosphate buffer, pH 7.2, 0,15 M NaCl, to which the fluorescently labeled antibodies (or conjugates of antibodies with peroxidase), specific to another epitope of the antigen, were added. After washing, the intensity of fluorescent or chemiluminescent signals was measured.

In certain cases conjugates of antibodies with biotin were used as the developing antibodies, and after incubation with biotinylated antibodies the microchip was developed with Cy3-labeled avidin or with the conjugate of avidin with peroxidase, this being followed by treatment with chemiluminescent substrates of peroxidase.

The antibodies, avidin and the antigens of protein nature, labeled with Cy 3 and Cy 5 fluorescent labels, and also the conjugates of antibodies and avidin with peroxidase were obtained by employing the known procedures (for example, described in G.T. Hermanson, Bioconjugate techniques, Academic Press, San Diego, 1996).

For measuring the fluorescent signals a fluorescent microscope was used, equipped with a CCD by camera and computer programs for the visualization of images and processing of obtained data, developed in the laboratory of biochips of the IMB RAS (V. Barsky, A. Perov, S. Tokalov, A. Chudinov, E. Kreindlin, A. Sharonov, E. Kotova, A. Mirzabekov, Fluorescence data analysis on of gel-based of biochips, J. Biomol. Screening, 2002, 7, 247-257). Microscope makes it possible to simultaneously analyze data from all elements of the microchip and to obtain two-dimensional and three-dimensional images of fluorescent signals from the gel elements.

For measuring the chemiluminescent signals the same fluorescent microscope with the turned-off source of excitation was used.

A graph of the dependence of the intensity of fluorescence or chemiluminescence on the concentration of antigen in solution was plotted for all variants of the assay. The smallest determined antigen concentration was calculated as the concentration corresponding to the signal intensity, 3 times exceeding the background signal spread.

### Example 2. Immunoassay of α-fetoprotein

Quantitative determination of AFP is necessary in diagnostics and treatment of a number of cancer diseases and also for revealing pregnancy pathologies. On the example of α-fetoprotein (AFP) we illustrate different variants of immunoassay with the use of protein hydrogel microchips - direct, competitive, and sandwich-type immunoassay with fluorescent detection **(Fig. 1)**.

For the direct assay, microchips with immobilized monoclonal antibodies against AFP were manufactured. After treating the microchips with fluorescently labeled AFP the dependence of the intensity of the fluorescence of the gel cells of the microchip on the concentration of the antigen was observed in the range of concentrations from 0.5 to 1000 ng/ml **(Fig. 1A),** the minimum determined AFP concentration was 0.5 ng/ml.

For the competitive assay, a microchip with immobilized AFP was used. The AFP solution was preliminarily incubated with Cy3-labeled monoclonal antibodies against AFP, and after incubation the solution was applied to the microchip. In this case the intensity of fluorescence was inversely proportional to the antigen concentration in the solution **(Fig. 1B).** A decrease of the fluorescent signal of the gel cells of the microchip, containing immobilized AFP, was observed as the AFP concentration in the solution varied from 2 to 2000 ng/ml.

In the case of sandwich immunoassay, a pair of monoclonal antibodies against different AFP epitopes was selected. Microchips with immobilized antibodies were manufactured. Developing antibodies were labeled with Cy3. The dependence of the intensity of fluorescence on the AFP concentration for the sandwich-type immunoassay on the chips is shown in **Fig. 1C.** The dependence of fluorescent signals on the AFP concentration was observed in the range of from 2 to 500 ng/ml. The minimally determined AFP concentration AFP was 2 ng/ml.

Under said conditions of carrying out the assay, in particular, with the procedures of blocking and washing the microchips, nonspecific signals, i.e. signals from the microchip cells, containing antibodies to other antigens (for instance, to other oncomarkers) practically did not differ from background signals or from the signals of empty gel elements. The immunoassay with the use of microchips is characterized by high sensitivity (Table 1) and reproducibility: the coefficient of variation in the measurements of the antigen concentration in the sample for all types of assay was not more than 10%.

**Table 1. Results of quantitative immunoassay of markers of malignant neoplasms on microchip**

| Marker (disease) | | Norm in blood | Pathological level | Detection threshold (sandwich-type immunoassay on microchips) | Detection threshold with the help of standard test systems |
|---|---|---|---|---|---|
| α-Fetoprotein (AFP) (carcinoma of the liver) | | 0 to 6.5 ng/ml (increased level is observed in the case of pregnancy and in newborns) | > 12.5 ng/ml | 2 ng/ml | 0.5 to 1 ng/ml |
| Prostate-specific antigen (PSA) (prostate cancer) | Total | 0 to 4 ng/ml | > 10 ng/ml | 0.05 ng/ml | 0.1 to 0.3 ng/ml |
| | Free | 0 to 1..3 ng/ml | - | 0.20 ng/ml | 0.03 to 0.1 ng/ml |
| Canceroembryonic antigen (CEA) (cancer of the gastrointestinal tract) | | 0 to 5 ng/ml | > 8 ng/ml | 0.5 ng/ml | 0.5 to 1 ng/ml |
| Cancer antigen 125 (CA 125 (ovarian cancer, cancer of uterine corpus) | | 0 to 30 MU/ml | > 40 MU/ml | 15 MU/ml | 1.5 to 5 MU/ml |
| Cancer antigen 19-9 (CA 19-9) (pancreatic carcinoma) | | 0 to 30 MU/ml | > 40 MU/ml | 2 MU/ml | 1 to 2 MU/ml |
| Cancer antigen 15-3 (CA 15-3) (cancer of the mammary gland) | | 0 t0 25 MU/ml | > 30 MU/ml | 4 MU/ml | 1 MUT/ml |

### Example 3. Chemiluminescent registration of a signal when carrying out immunoassay with use of microchips

Microchips with immobilized monoclonal antibodies against horseradish peroxidase have been manufactured. 20 µl of peroxidase solutions of different concentrations in 0.01 M phosphate buffer, pH 7.2, containing 0.15 M NaCl were applied to the microchips, microchips were kept for 2 hours at room temperature, and then 20 µl of a mixture of chemiluminescent substrates (SuperSignal ELISA Pico Stable Peroxide, Luminol Enhancer, Pierce Corp., USA), containing luminol, hydrogen peroxide and the enhancer. During peroxidase-catalyzed oxidation of luminol, chemiluminescence with a maximum at 425 nanometers is observed. For registration of the signals a fluorescence microscope was used with the excitation light lamp switched-off, the exposure was 60 s. The measured intensity of the chemiluminescence of gel cells containing immobilized antibodies to peroxidase, was directly proportional both to concentrations of immobilized antibodies, and peroxidase concentrations in solution in a range of 0.5-500 ng/ml **(Figs.** 2)**.** Thus, it is shown, that chemiluminescent detection can be used for the registration of immunoassay results on the microchips, and the sensitivity of this detection is not inferior to detection with the use of a fluorescent label.

### Example 4. Immunoassay of markers of oncological diseases with the use of microchips

In Table 1 and in **Fig. 3** the results of quantitative immunoassay of markers of oncological diseases using gel microchips are presented. In addition to the immunoassay of α-fetoprotein, described in the Example 1, an analysis of prostate-specific antigen (total and free forms), canceroembryonic antigen (CEA), cancer antigens CA 125, CA 15-3 and CA 19-9 was carried out. A direct, competitive and a sandwich-type immunoassay with fluorescent and chemiluminescent registration were carried out by following the procedures described in Example 1. In **Figure 4** a microchip is shown for sandwich-type immunoassay of prostate-specific antigen (PSA), containing gel elements with immobilized PSA for plotting calibration curve.

### Example 5. Simultaneous analysis of several antigens on one microchip

Microchips were manufactured with immobilized antibodies to all investigated markers of oncological diseases (α-fetoprotein (AFP), prostate-specific antigen (PSA, total and free forms), canceroembryonic antigen (CEA), cancer antigens CA 125, CA 19-9, CA 15-3 (**Fig**. 3). After incubation of a microchip with solution or blood serum containing the analyzed antigen, the microchip was developed with a mixture of fluorescently labeled antibodies to all oncomarkers. As is seen from **Fig. 3**, bright signals were observed only in the gel cells containing specific antibodies. The limits of detecting oncomarkers in the case of their parallel analysis have appeared to be the same as in detecting each marker separately (Table 1).

### Example 6. Immunoassay of total immunoglobulin E in blood serum on a microchip

The microchip for immunoassay of total IgE contained immobilized antibodies to human immunoglobulins E (anti-IgE). After the incubation with blood serum the microchip was developed with second fluorescently labeled anti-IgE antibodies. **Fig 5** shows a calibration curve for determination of the total IgE in a range of 25-400 MU/ml in double logarithmic coordinates.

### Example 7. Immunoassay specific immunoglobulin E (timothy grass allergen) in blood serum on a microchip

For analysis of specific IgE the microchip contained immobilized allergen (purified protein fraction of an allergen from timothy grass). After incubation with human blood serum the microchip was developed with fluorescently labeled anti-IgE as in the case of determining total IgE. Shown in **Fig. 6** are fluorescent images of the microchip after the interaction with blood serum of a patient with a high content of specific IgE (++++ according to the skin test analysis), with the average content of specific IgE (+ + according to the skin test analysis) and with blood serum of healthy donor (no specific IgE are present).

### Example 8. Immunoassay of monoclonal antibodies against trisaccharides A and B

Microchips contained trisaccharides A and B (agglutinogens of red cells of the blood, determining the group of human blood), immobilized in gel elements. The concentration of trisaccharides in the gel was 1 mg/ml. The microchips were incubated with solutions of human serum of blood group III (contains antibodies only against trisaccharide A) and of human serum of blood group II (contains antibodies only against trisaccharide B) in various dilutions, overnight at +4°C. Oligosaccharide microchips were washed in a 0.01 M phosphate buffer, pH 7.2, 0.15 M NaCl, for 20 min and then treated with a solution of murine antibodies IgM against human antibodies, 0.01 mg/ml, overnight at +4°C, washed the same buffer solution during 20 min, then treated with a solution of goat antibodies against murine IgM, stained with Cy5, 0.01 mg/ml, overnight at +4°C. After washing in a 0.01 M phosphate buffer, pH 7.2, 0.15 M NaCl, 0.1% Tween-20, 20 min, fluorescent signals of the gel cells were measured by means of a fluorescence microscope. The dependence of the intensity of fluorescence on the dilution of blood serum containing antibodies against trisaccharides A and B was plotted (**Fig. 7**). The gel oligosaccharide microchips have allowed to register the presence of antibodies to trisaccharides A and B at the dilution of blood serum to 1:10000 (standard method of ELISA assay- at the 1:500 dilution).

### Example 9. Immunoassay acceleration with the use of sample agitation

The sandwich-type immunoassay of prostate-specific antigen (PSA) was carried out on microchips with immobilized monoclonal antibodies against PSA, 0.1 mg/ml. The microchips were incubated in the reaction medium containing simultaneously PSA and monoclonal antibodies against other PSA epitope, labeled with Cy3 (20 µg/ml) during 2 hours at room temperature with agitation and without agitation. Agitation was carried out by means of a peristaltic pump. For this purpose a microchip was placed in a flow chamber 50 µl in volume, equipped with pipes for admission of the sample. The chamber was coupled to a peristaltic pump Minipuls 2 (Gilson), and 70 µl the sample to be analyzed, containing PSA, was placed into the system. The sample was agitated by switching the direction of flow from direct to reverse and vice versa every 2 seconds; the rate of the flow was 2 ml/min. Simultaneously the analysis with agitation was carried out on 6 microchips, i.e. measurement of 6 samples with various concentrations of PSA. In **Fig. 8** calibration curves are shown for the determination of PSA in the case of sandwich-type immunoassay with agitation of the sample (curve 1) and without agitation (curve 2). After 2 hours of agitation the intensity of fluorescence signal from the gel elements of the microchip, containing immobilized antibodies, was higher, than without agitation for all concentrations of PSA in solution: for PSA in solution of 15-125 ng/ml there was a 5-fold signal amplification and greater, for PSA in solution of 250 ng/ml and more the amplification was 4-fold. The intensity of background signals (empty gel elements) did not vary.

### Example 10. Immuno-PCR on gel microchips

The immuno-PCR on gel microchips was carried out with the use of a conjugate of an antibody with an oligonucleotide as the developing antibody. A conjugate of monoclonal antibodies against horseradish peroxidase with an oligonucleotide having the following sequence: **Ab-S-(C6)-CAG CTT AAT ACG ACT CAC** TAT **AGG GAC CGT CAG TGC GCG ATT AAT-OH-3'** was synthesized. The synthesis of the conjugate was carried out according to the procedure described in G.T. Hermanson, Bioconjugate Techniques, Academic Press, San Diego, 1996, with use of 3-sulfo-N-hydroxysuccinimide ester of 4-(N-maleinimidomethyl)-cyclohexane-1-carboxylic acid (sulfo-SMCC).

Microchips were manufactured, each of which contained gel elements with immobilized peroxidase, elements with oligonucleotides, completely complementary to the fluorescently labeled product produced as a result of the CPR, non-complementary oligonucleotides and empty gels (**Fig. 9A**). Complementary oligonucleotide - **5'-NH₂-(C₆) CTA TAG GGA CCG TCA-OH-3 ';** Non-complementary oligonucleotides - **5'-NH₂(C₆) GAT GCT GAC TAA CGC-OH-3 '.**

Concentration of the immobilized oligonucleotides on the microchip was 200 picomol/µl gel, the concentration of the peroxidase immobilized in the gel was 0.1 mg/ml. The microchips were washed for 20 min in a 0.01 M phosphate buffer, pH 7.2, containing 0.15 M NaCl, 0.1 % Tween-20. The microchips were treated with a blocking solution (0.01 M phosphate buffer, pH 7,2, 0.15 M NaCl, 0.01 % PVA) during 1 hour and then incubated with the conjugate of antibodies with an oligonucleotide in different concentrations (1-1000 ng/ml in 0.01 M phosphate buffer, pH 7.2, 0.15 M NaCl, 0.15 % PVA and 0.15 % PVP) within 2.5 hours at room temperature. After incubation the microchips were washed for 20 min in 0.01 M phosphate buffer, pH 7.2. 0.15 M NaCl, 0.1% Tween-20, 20 µl of a mixture for the PCR were applied to the chip in a chamber 25 µl in volume (Eppendorf In situ Frame), and the chip was placed in the apparatus for carrying out PCR (TGradient Thermocycler, Biometra GmbH). asymmetric PCR was carried out on the microchip with primers **Primer 1 - 5'-CAG CTT AAT ACG ACT CA-OH-3'** and fluorescently labeled **Primer 2 - 3'-TC ACG CGC TAA TTA-Texas Red-5'.**

The composition of the mixture for the PCR (all reagents from the «DNA amplification» of the « Silex M », Russia) comprised:

| | |
|---|---|
| 1. Sterile water | 9,6 µl; |
| 2 Reaction buffer (x 10) | 4 µl; |
| 3. MgCl₂ (25 Mm) | 2 µl; |
| 4. dNTP mixture (1.5 Mm) | 2 µl: |
| 5. Primer 1 (0.2 µM) | 1 µl; |
| 6. Primer 2 (10 µM) | 1 µl; |
| 7. Taq-polymerase | 0.4 µl. |

Final concentrations of the primers in the mixture were 0.01 µM (Primer 1) and 0.5 µM (Primer 2), the Primer 1: Primer 2 ratio = 1:50.

**Mode of carrying out PCR:**

| | | |
|---|---|---|
| T=94° | 2 min; | |
| T=94° | 15 s; | |
| T-50° | 20 s; | 30 cycles |
| T=72° | 20 s; | |
| T=10. | | |

As a result of the PCR fluorescently labeled oligonucleotide is produced, complementary to the initial matrix. On completion of the PCR the chips were washed during 5 min with 0.01 M phosphate buffer, pH 7.2, 0.15 M NaCl, 0.1% Tween-20, and measurements were carried out on the fluorescence microscope.

Concurrently hybridization of the fluorescently labeled oligonucleotide, complementary to the matrix (Primer 2), with an oligonucleotide immobilized on a microchip under usual conditions was carried out on microchips: after incubation with the antibody-oligonucleotide conjugate, a solution of Primer 2 was applied to the microchip in concentration 0.5 µM in the 0.05 M phosphate buffer, pH 7.2, 0.15 M NaCl, and microchip was incubated during 1.5 hours at room temperature. The microchip was washed for 5 min with the 0.01 M the phosphate buffer, pH 7.2, 0.15 M NaCl, 0.1% Tween-20, and measurements were carried out on a fluorescence microscope.

**Fig. 9B** shows fluorescent image of the microchip after immuno-PCR with the solution containing 1 ng/ml of the conjugate. Bright signals from the cells with the immobilized complementary oligonucleotides were observed. In usual hybridization of the microchip with an oligonucleotides complementary to the matrix, labeled with Texas red fluorescent dye (Primer 2), at the conjugate concentration of 1 ng/ml fluorescent signals were not observed (**Fig. 9C**). The fluorescent signal in usual hybridization was observed only at the incubation of a microchip with solution of an antibody-oligonucleotide conjugate at the concentration of 500 ng/ml, and the intensity of fluorescence in that case was lower than in the case of carrying out Immuno-PCR with the conjugate in the concentration of 1 ng/ml.

Thus, in carrying out immuno-PCR on a microchip, the signal is intensified approximately 1000-fold. One more important and unexpected advantage of carrying out PCR directly on a microchip is that the antibody-oligonucleotide conjugate after its synthesis can be used without additional purification thereof When carrying out immuno-PCR on immunological plates (E.R. Hendrickson, T.M. Truby, R.D. Joerger, W.R. Marjarian, R.C. Ebersole, High sensitivity multianalyte immunoassay using covalent DNA-labeled antibodies and polymerase chain reaction, Nucleic Acids Res., 1995, 23, 522-529) the obtained conjugates were subjected to multistage purification with the use of various chromatographic methods. Microchip in the given case operates as an affine column on which only a specific binding takes place, and extraneous materials are removed during washings.

### Example 11. Identification of compounds on gel element of a microchip by direct mass-spectral analysis

Microchips containing immobilized monoclonal antibodies against insulin and lysozyme (0.1 µg of antibodies/gel cell) in gel cells were manufactured. After polymerization the microchip was washed with 0.01 M phosphate buffer, 0.15 M NaCl, 0.1 % Tween-20, with agitation during 1 hour (20°C). The microchip was incubated with the solution containing insulin or lysozyme in 0.01 M phosphate buffer, pH 7.2, 0.15 M NaCl (20 hours, 20°C), and further washed with 0.01 M phosphate buffer, pH 72, 0.15 M NaCl, 0.1 % Tween-20 (2 hours with agitation, 20°C), and then with water. Before carrying out mass-spectral analysis the antigen-antibody complex was destroyed and the antigen was eluted to the gel surface by adding to each cell of the microchip 1 µl the saturated solution of a template for MALDI monitoring (sinapinic acid) in solution of 10% formic acid 30% aqueous acetonitrile. The microchip was kept for 20 min at room temperature in a humidified chamber, then the slide was dried on a heating stage at 40°C, and mass-spectrometric measurements were carried out. For control, fluorescent measurements with the use of insulin, labeled with Bodipy fluorescent dye were concurrently carried out: before carrying out mass-spectral analysis a signal was obtained from the given cell of the microchip by means of fluorescence microscope. In the case of biotinylated insulin for the fluorescent measurement the biochip was treated with solution of fluorescently labeled avidin.

Mass-spectra obtained directly from the gel elements of the microchip, after interaction with the solutions of insulin-Bodipy and biotinylated lysocim are shown in **Fig. 10**. The spectra contained peaks corresponding to the molecular masses of the given compounds. The mass-spectra from the gel cells, which did not contain antibodies against these insulin and lysocim, but were incubated with the solution of these compounds, did not show the above-mentioned peaks. In the concurrent fluorescent measurements only specific signals were also obtained.

### Example 12. Proteolytic hydrolysis in gel elements of a microchip

A microchip according to Example 1 was manufactured with a volume of the gel cells of 0.2 µl, containing cells with immobilized lysozyme (0.5 µg lysozyme/gel cell). After polymerization the chip was washed with 0.01 M phosphate buffer, 0.15 M NaCl, 0.1% Tween-20, with agitation during 2 hours and then with water. For the denaturation and breakage of S-S-bonds of proteins, 1 µl of denaturizing buffer (50 mM tris-HCl, pH 8.0, 5 mM dithiothreitol, 6 M guanidine chloride) into each gel cell of the microchip was added and the microchip was incubated in a moist chamber at 65°C during 4 hours. After that the microchip was washed quickly with 0.2 M ammonium bicarbonate, heated to 65°C. The chip was desiccated dry at 37°C on a heating stage, and a solution of trypsin in 0.2 M ammonium bicarbonate (25 ng of trypsin in 1 µl of the bicarbonate buffer, the protease/: immobilized protein ratio 1 : 20) was gradually added to dry gel cells. The microchip was incubated in the moist chamber at 37°C during 20-24 hours. The reaction was interrupted by adding 0.1% aqueous solution of trifluoroacetic acid (TFA) to each cell of the gel. The microchip was desiccated dry at 37°C.

For the MALDI MS analysis, 1 µl of 35 % solution of acetonitrile in 0.1% TFA was added to each gel cell, the microchip was dried on the heating stage at 40°C, and mass-spectra were obtained directly from the gel cells of the microchip **(Fig.** 11). Concurrently mass-spectral analysis was carried out from the microchip cells which did not contain immobilized protein, after the addition of the same quantity of trypsin. From the mass-spectra of trypsin hydrolyzates of protein (lysozyme) in the gel, the mass-spectra of hydrolyzed trypsin were subtracted. The results of mass-spectral analysis of the trypsin hydrolysate of lysozyme directly from the gel cell of the microchip minus the hydrolysate of the very trypsin are presented in Table 2.

**Table 2. Mass spectrum of tryptic hydrolysate of lysozyme, obtained from microchip gel element on COMPACT MALDI 4 mass spectrometer (Kratos analytical)**

| Number of peptide in the sequence | Measured molecular mass of peptide | Theoretically calculated mass of peptide | Structure of peptide |
|---|---|---|---|
| 147-147 | - | 131.2 | L |
| 115-115 | - | 146.2 | K |
| 19-19 | - | 146.2 | K |
| 32-32 | - | 174.2 | R |
| 133-134 | - | 249.3 | CK |
| 131-132 | - | 288.3 | NR |
| 144-146 | 335.0 | 334.4 | GCR |
| 20-23 | 480.0 | 477.6 | VFGR |
| 87-91 | 520.3 | 516.5 | TPGCR |
| 131-134* | 520.3* | 519.6* | NRCK* |
| - | 727.6** | - | - |
| 24-31 | 838.0 | 836.0 | CELAAAMK |
| 33-39 | 876.0 | 873..9 | HGLDNYR |
| 80-86 | 937.3 | 936.0 | WWCNDGR |
| 135-143 | 1046.6 | 1045.2 | GTDVQAWIR |
| 40-51 | - | 1268.5 | GYSLGNWVCAAK |
| 133-143* | 1278.5* | 1276.5* | CKGTDVQAWIR* |
| 52-65 | 1430.9 | 1428.5 | FESNFNTQATNR |
| 116-130 | 1676.9 | 1675.9 | IVSDGNGMNAWVAWR |
| 92-114 | 2323.2 | 2337.7 | NLCNIPCSALLSSDITASVNCAK |
| 92-115* | 2459.7* | 245.9* | NLCNIPCSALLSSDITASVNCAKK |
| 87-114* | 2814.1* | 2836.0* | TPGCRNLCNIPCSALLSSDITASVNCAK |

| | | | |
|---|---|---|---|
| *) Peptides formed in incomplete hydrolysis of lysozyme by trypsin (1 omitted site); **) Unidentified peak. | | | |

The amino acid sequence of lysozyme consists of 128 residues with Nos. 19-147 (the residues with Nos. 1-18 belong to the signal sequence). Complete hydrolysis of lysozyme by trypsin leads to the formation of 18 peptides, six of them consist of one or two residues with masses less than 300 which are not registered mass-spectrally under the given conditions. Theoretically calculated molecular weights of the peptides after the hydrolysis of lysozyme by trypsin, obtained from SWISS-PROT protein database (http: /cn.expasy.org/cgi-bin/peptide-mass/pl) are also shown in Table 2. The mass spectrum of the lysozyme hydrolyzed by trypsin in the gel cell of the microchip contained 14 peaks, 10 of which correspond to the peptides, formed in complete hydrolysis, 3 peaks corresponding to peptides of incomplete hydrolysis with one omitted site. The peak with the molecular weight 520.3 can correspond both to peptide 87-91 with molecular weight 516.5 for the complete hydrolysis by trypsin, and to peptide 131-134 with the molecular weight 519.6, formed in the hydrolysis with one omitted site. The peak with the molecular weight 727.6 was not identified.

The obtained mass spectral data were introduced into the Peptldent program from database SWISS-PROT database for the analysis of "peptide fingerprints". The search was carried out among proteins with molecular weight in a range of 12880-15750, for the case of hydrolysis by trypsin, the accuracy of determination of the weights was ± 5 Da, the cystein residues were in reduced form. The result: for the cited molecular weights of the tryptic hydrolysate with an accuracy of 75 % the protein has been identified as chicken lysozyme (Gallus gallus) with molecular weight 14313 Da.

All the publications and patent documents mentioned in the description are included by reference in full scope thereof. To one skilled in the art it is clear that various changes and modifications can be made without going beyond the framework of the present invention as defined by the following set of claims.

## Claims

1. A biological microchip for multiple parallel immunoassay of compounds, comprising an array of three-dimensional hydrogel elements of a prescribed volume, formed on a support by the method of photo- or chemically induced polymerization, and containing biological molecules of the same or different nature (ligands), and permitting sequential carrying out reactions of different type.

2. The biological microchip according to claim 1, **characterized in that** the hydrogel element contains an immobilized ligand of protein or non-protein nature, which during the microchip incubation in the reaction medium including a sample comprising compounds subject to assay forms a specific immune complex of "antigen-antibody" type.

3. The biological microchip according to claim 1, **characterized in that** the ligands in the hydrogel cells are selected from the group comprising antibodies of any type and antigens of various nature.

4. The biological microchip according to claim 1, **characterized in that** the compounds subject to analysis are compounds which are selected from the group comprising tumor-associated antigens - markers of oncological diseases, total human immunoglobulin E, specific antibodies (human immunoglobulins E) against different allergens, polysaccharides and specific antibodies against polysaccharides.

5. The biological microchip according to claim 1, **characterized in that** for the immunoassay of tumor-associated antigens - markers of oncological diseases antibodies to said tumor-associated antigens or the tumor-associated antigens as such are used as the immobilized ligands.

6. The biological microchip according to claim 1, **characterized in that** for the immunoassay of total human immunoglobulin E antibodies against human immunoglobulins E or human immunoglobulins E are used as the immobilized ligands.

7. The biological microchip according to claim 1, **characterized in that** for the immunoassay of specific antibodies to different allergens different allergens are used as the immobilized ligands.

8. The biological microchip according to claim 1, **characterized in that** for the immunoassay of polysaccharides or specific antibodies against polysaccharides different polysaccharides and/or antibodies against polysaccharides are used as the immobilized ligands.

9. A method of detection of compounds on the biological microchip **characterized in** claim 1, comprising identification of said compounds by the method of mass spectrometry, **characterized in that** the identification is carried out directly on the hydrogel element of the microchip according to the results of direct mass-spectral analysis of the compound bound to the ligand.

10. A method of detection of compounds of protein nature on the biological microchip **characterized in** claim 1, comprising identification of said compounds by the method of mass spectrometry, **characterized in that** the identification is carried out directly on the hydrogel element of the microchip according to the results of direct mass-spectral analysis of the protein bound to the ligand or of peptides obtained by carrying out proteolytic hydrolysis of the protein bound to the ligand directly on the hydrogel element of the microchip.

11. A method of multiple immunoassay of compounds on the biological microchip **characterized in** claim 1, contemplating:
*a)* incubation of the biological microchip in a reaction medium comprising a sample containing compounds subject to analysis for forming immune complexes with the ligands immobilized on the microchip, which incubation, if necessary, is carried out under agitation conditions;
*b)* detection of the formed complex;
*c)* if necessary, quantitative determination of the compound subject to analysis.

12. The method according to claim 11, comprising an immunoassay of any type (direct, indirect, competitive, sandwich-type immunoassay, and others).

13. The method according to claim 11, in which for the quantitative determination in step *c*), steps *a)-b)* are carried out with the known concentrations of the compound subject to analysis and a calibration dependence is plotted to determine the content of the compound under analysis.

14. The method according to claim 11, in which in step *b)* the detection of the formed complex is carried out fluorimetrically, chemiluminometrtically, mass-spectrometrically or by carrying out immuno-CPR followed by registering the CPR results on the same microchip.

15. A method of multiple parallel immunoassay of oncomarkers on the biological microchip **characterized in** claim 1, containing immobilized antibodies against oncomarkers and/or immobilized oncomarkers, contemplating;
*a)* incubation of the biological microchip in a reaction medium comprising a sample containing oncomarkers subject to analysis for forming an immune complex with the ligands immobilized on the microchip, which incubation, if necessary, is carried out under agitation conditions;
*b)* detection of the formed complex;
*c*) if necessary, quantitative determination of the oncomarker subject to analysis.

16. The method according to claim 15, comprising an immunoassay of any type (direct, indirect, competitive, sandwich-type immunoassay, and others).

17. The method according to claim 15, in which for the quantitative determination in step *c*), steps *a)-b)* are carried out with the known concentrations of the oncomarker subject to analysis and a calibration dependence is plotted to determine the content of the oncomarker under analysis in the sample.

18. The method according to claim 15, in which in step *b)* the detection of the formed complex is carried out fluorimetrically, chemiluminometrtically, mass-spectrometrically or by carrying out immuno-CPR followed by registering the CPR results on the same microchip.

19. A method of multiple parallel immunoassay of total human immunoglobulin E on the biological microchip **characterized in** claim 1, containing immobilized antibodies against human immunoglobulin E (anti-IgE) or immobilized human immunoglobulins E (IgE), contemplating:
*a)* incubation of the biological microchip in a reaction medium comprising a sample containing total human immunoglobulins subject to analysis for forming an immune complex with the ligands immobilized on the microchip, which incubation, if necessary, is carried out under agitation conditions;
*b*) detection of the formed complex;
c) if necessary, quantitative determination of the total human immunoglobulin E subject to analysis.

20. The method according to claim 19, comprising an immunoassay of any type (direct, indirect, competitive, sandwich-type immunoassay, and others).

21. The method according to claim 19, in which for the quantitative determination in step *c),* steps *a)-b)* are carried out with the known concentrations of the total human immunoglobulin subject to analysis and a calibration dependence is plotted to determine the content of the total human immunoglobulin E under analysis in the sample.

22. The method according to claim 19, in which in step c) the detection of the formed complex is carried out fluorimetrically, chemiluminometrtically, mass-spectrometrically or by carrying out immuno-CPR followed by registering the CPR results on the same microchip.

23. A method of multiple parallel immunoassay of allergen-specific immunoglobulins E on the biological microchip **characterized in** claim 1, containing immobilized specific allergens, contemplating:
*a)* incubation of the biological microchip in a reaction medium comprising a sample containing allergen-specific IgE subject to analysis for forming an allergen - allergen-specific antibody complex, and the incubation, if necessary, is carried out under agitation conditions;
*b)* detection of the formed complex;
*c)* if necessary, quantitative determination of the allergen-specific IgE subject to analysis.

24. The method according to claim 23, comprising an immunoassay of any type (direct, indirect, competitive, sandwich-type immunoassay, and others).

25. The method according to claim 23, in which for the quantitative determination in step *c),* steps *a)-b)* are carried out with the known concentrations of the allergen-specific IgE and a calibration dependence is plotted to determine the content of the allergen-specific IgE under analysis in the sample.

26. The method according to claim 23, in which in step *b)* the detection of the formed complex is carried out fluorimetrically, chemiluminometrtically, mass-spectrometrically or by carrying out immuno-CPR followed by registering the CPR results on the same microchip.

27. A method of multiple parallel immunoassay against polysaccharides on the biological microchip **characterized in** claim 1, containing immobilized polysaccharides or antibodies against polysaccharides, contemplating:
*a)* incubation of the biological microchip in a reaction medium comprising a sample containing antibodies against polysaccharides subject to analysis for forming a polysaccharide-antibody complex, which incubation, if necessary, is carried out under agitation conditions;
*b*) detection of the formed complex;
c) if necessary, quantitative determination of the antibodies against polysaccharides or polysaccharides.

28. The method according to claim 27, wherein the immobilized polysaccharides are trisaccharides A and B - agglutinogens of red blood cells, which determine the group of human blood.

29. The method according to claim 27, wherein for the quantitative determination in step *c*), steps *a)-b)* are carried out with the known concentrations of the antibody against polysaccharide under analysis and a calibration dependence is plotted to determine the content of the antibody against polysaccharide subject to analysis in the sample.

30. The method according to claim 27, comprising an immunoassay of any type (direct, indirect, competitive, sandwich-type immunoassay, and others).

31. The method according to claim 27, wherein in step *b)* the detection of the formed complex is carried out fluorimetrically, chemiluminometrically, mass-spectrometrically or by carrying out the immuno-PCR , followed by registration of the PCR results on the same microchip.

32. A method of multiple parallel immunoassay of polysaccharides on the biological microchip **characterized in** claim 1, containing immobilized polysaccharides or antibodies against polysaccharides, contemplating:
*a)* incubation of the biological microchip in a reaction medium comprising a sample containing polysaccharides subject to analysis for forming a polysaccharide-antibody complex, which incubation, if necessary, is carried out under agitation conditions;
*b)* detection of the formed complex;
*c)* if necessary, quantitative determination of the antibodies against polysaccharides or polysaccharides.

33. The method according to claim 32, wherein for the quantitative determination in step *c)*, steps *a)*-*b)* are carried out with the known concentrations of the polysaccharide under analysis and a calibration dependence is plotted, using which the content of the polysaccharide under analysis in the sample is determined.

34. The method according to claim 32, comprising an immunoassay of any type (direct, indirect, competitive, sandwich-type immunoassay, and others).

35. The method according to claim 32, wherein in step *b)* the detection of the formed complex is carried out fluorimetrically, chemiluminometrically, mass-spectrometrically or by carrying out the immuno-PCR , followed by registration of the PCR results on the same microchip.
